# EUROPEAN PATENT APPLICATION

(11) **EP 2 960 248 A1**
(43) Date of publication of application: **30.12.2015**
(21) Application number: 14754342.5
(22) Date of filing: 25.02.2014
(51) Int. Cl.: C07K 1/16, C07K 19/00, C07K 14/745

(54) **METHOD OF ISOLATING AND PURIFYING FUSION PROTEIN COMPRISING FACTOR VII**

(30) Priority: 25.02.2013 KR 20130019913
(71) Applicant: SK Chemicals Co., Ltd., Seongnam-Si, Gyeonggi-Do 463-400 (KR)
(72) Inventor: LEE, Ji-Hye, Seongnam-si Gyeonggi-do 463-876 (KR); KANG, Seok-chan, Bucheon-si Gyeonggi-do 420-752 (KR); RYU, Yangkyun, Seoul 152-773 (KR); LEE, Ho Soon, Seongnam-si Gyeonggi-do 463-977 (KR); SONG, In-Young, Seoul 156-773 (KR); KIM, Hun-Taek, Seoul 137-796 (KR)
(74) Representative: Jones, Elizabeth Louise
(86) International application number: PCT/KR2014/001526
(87) International publication number: WO 2014/129876

(57) **Abstract**

The present invention provides a method of isolating and purifying a fusion protein comprising factor VII, and more specifically relates to a method of isolating and purifying a fusion protein comprising factor VII and transferrin, to a high degree of purity. Because the present invention provides a method whereby a recombinant fusion protein comprising factor VII can be isolated and purified to a high degree of purity, the invention is useful in producing a pharmaceutical preparation comprising factor VII that can be used in situations in which copious bleeding occurs such as surgery.

SEQUENCE LISTING

<110>    SK CHEMICALS CO., LTD.

<120>    METHOD FOR ISOLATING AND PURIFYING A FUSION PROTEIN COMPRISING
         FACTOR VII

<130>    PCB402004

<150>    10-2013-0019913
<151>    2013-02-25

<160>    53

<170>    KopatentIn 1.71

<210>    1
<211>    444
<212>    PRT
<213>    Homo sapiens

<220>
<221>    PEPTIDE
<222>    (1)..(444)
<223>    Factor VII protein

<400>    1
Met Val Ser Gln Ala Leu Arg Leu Leu Cys Leu Leu Leu Gly Leu Gln
1               5                   10                  15

Gly Cys Leu Ala Ala Val Phe Val Thr Gln Glu Glu Ala His Gly Val
            20                  25                  30

Leu His Arg Arg Arg Arg Ala Asn Ala Phe Leu Glu Glu Leu Arg Pro
        35                  40                  45

Gly Ser Leu Glu Arg Glu Cys Lys Glu Glu Gln Cys Ser Phe Glu Glu
    50                  55                  60

Ala Arg Glu Ile Phe Lys Asp Ala Glu Arg Thr Lys Leu Phe Trp Ile
65                  70                  75                  80

Ser Tyr Ser Asp Gly Asp Gln Cys Ala Ser Ser Pro Cys Gln Asn Gly
                85                  90                  95

Gly Ser Cys Lys Asp Gln Leu Gln Ser Tyr Ile Cys Phe Cys Leu Pro
            100                 105                 110

Ala Phe Glu Gly Arg Asn Cys Glu Thr His Lys Asp Asp Gln Leu Ile
        115                 120                 125

Cys Val Asn Glu Asn Gly Gly Cys Glu Gln Tyr Cys Ser Asp His Thr
    130                 135                 140

Gly Thr Lys Arg Ser Cys Arg Cys His Glu Gly Tyr Ser Leu Leu Ala
145                 150                 155                 160

Asp Gly Val Ser Cys Thr Pro Thr Val Glu Tyr Pro Cys Gly Lys Ile
                165                 170                 175

Pro Ile Leu Glu Lys Arg Asn Ala Ser Lys Pro Gln Gly Arg Ile Val
            180                 185                 190

Gly Gly Lys Val Cys Pro Lys Gly Glu Cys Pro Trp Gln Val Leu Leu
        195                 200                 205

Leu Val Asn Gly Ala Gln Leu Cys Gly Gly Thr Leu Ile Asn Thr Ile

210                 215                 220

Trp Val Val Ser Ala Ala His Cys Phe Asp Lys Ile Lys Asn Trp Arg
225                 230                 235                 240

Asn Leu Ile Ala Val Leu Gly Glu His Asp Leu Ser Glu His Asp Gly
                245                 250                 255

Asp Glu Gln Ser Arg Arg Val Ala Gln Val Ile Ile Pro Ser Thr Tyr
            260                 265                 270

Val Pro Gly Thr Thr Asn His Asp Ile Ala Leu Leu Arg Leu His Gln
        275                 280                 285

Pro Val Val Leu Thr Asp His Val Val Pro Leu Cys Leu Pro Glu Arg
    290                 295                 300

Thr Phe Ser Glu Arg Thr Leu Ala Phe Val Arg Phe Ser Leu Val Ser
305                 310                 315                 320

Gly Trp Gly Gln Leu Leu Asp Arg Gly Ala Thr Ala Leu Glu Leu Met
                325                 330                 335

Val Leu Asn Val Pro Arg Leu Met Thr Gln Asp Cys Leu Gln Gln Ser
            340                 345                 350

Arg Lys Val Gly Asp Ser Pro Asn Ile Thr Glu Tyr Met Phe Cys Ala
        355                 360                 365

Gly Tyr Ser Asp Gly Ser Lys Asp Ser Cys Lys Gly Asp Ser Gly Gly
    370                 375                 380

Pro His Ala Thr His Tyr Arg Gly Thr Trp Tyr Leu Thr Gly Ile Val
385                 390                 395                 400

Ser Trp Gly Gln Gly Cys Ala Thr Val Gly His Phe Gly Val Tyr Thr
                405                 410                 415

Arg Val Ser Gln Tyr Ile Glu Trp Leu Gln Lys Leu Met Arg Ser Glu
            420                 425                 430

Pro Arg Pro Gly Val Leu Leu Arg Ala Pro Phe Pro
        435                 440

<210>   2
<211>   679
<212>   PRT
<213>   Homo sapiens

<220>
<221>   PEPTIDE
<222>   (1)..(679)
<223>   Transferrin protein

<400>   2
Val Pro Asp Lys Thr Val Arg Trp Cys Ala Val Ser Glu His Glu Ala
1               5                   10                  15

Thr Lys Cys Gln Ser Phe Arg Asp His Met Lys Ser Val Ile Pro Ser
            20                  25                  30

Asp Gly Pro Ser Val Ala Cys Val Lys Lys Ala Ser Tyr Leu Asp Cys
        35                  40                  45

Ile Arg Ala Ile Ala Ala Asn Glu Ala Asp Ala Val Thr Leu Asp Ala
    50                  55                  60

Gly Leu Val Tyr Asp Ala Tyr Leu Ala Pro Asn Asn Leu Lys Pro Val
65                  70                  75                  80

Val Ala Glu Phe Tyr Gly Ser Lys Glu Asp Pro Gln Thr Phe Tyr Tyr
                85                  90                  95

Ala Val Ala Val Val Lys Lys Asp Ser Gly Phe Gln Met Asn Gln Leu
            100                 105                 110

Arg Gly Lys Lys Ser Cys His Thr Gly Leu Gly Arg Ser Ala Gly Trp
        115                 120                 125

Asn Ile Pro Ile Gly Leu Leu Tyr Cys Asp Leu Pro Glu Pro Arg Lys
    130                 135                 140

Pro Leu Glu Lys Ala Val Ala Asn Phe Phe Ser Gly Ser Cys Ala Pro
145                 150                 155                 160

Cys Ala Asp Gly Thr Asp Phe Pro Gln Leu Cys Gln Leu Cys Pro Gly
                165                 170                 175

Cys Gly Cys Ser Thr Leu Asn Gln Tyr Phe Gly Tyr Ser Gly Ala Phe
            180                 185                 190

Lys Cys Leu Lys Asp Gly Ala Gly Asp Val Ala Phe Val Lys His Ser
        195                 200                 205

Thr Ile Phe Glu Asn Leu Ala Asn Lys Ala Asp Arg Asp Gln Tyr Glu
    210                 215                 220

Leu Leu Cys Leu Asp Asn Thr Arg Lys Pro Val Asp Glu Tyr Lys Asp
225                 230                 235                 240

Cys His Leu Ala Gln Val Pro Ser His Thr Val Val Ala Arg Ser Met
                245                 250                 255

Gly Gly Lys Glu Asp Leu Ile Trp Glu Leu Leu Asn Gln Ala Gln Glu
            260                 265                 270

His Phe Gly Lys Asp Lys Ser Lys Glu Phe Gln Leu Phe Ser Ser Pro
        275                 280                 285

His Gly Lys Asp Leu Leu Phe Lys Asp Ser Ala His Gly Phe Leu Lys
    290                 295                 300

Val Pro Pro Arg Met Asp Ala Lys Met Tyr Leu Gly Tyr Glu Tyr Val
305                 310                 315                 320

Thr Ala Ile Arg Asn Leu Arg Glu Gly Thr Cys Pro Glu Ala Pro Thr
                325                 330                 335

Asp Glu Cys Lys Pro Val Lys Trp Cys Ala Leu Ser His His Glu Arg
            340                 345                 350

Leu Lys Cys Asp Glu Trp Ser Val Asn Ser Val Gly Lys Ile Glu Cys
        355                 360                 365

Val Ser Ala Glu Thr Thr Glu Asp Cys Ile Ala Lys Ile Met Asn Gly
    370                 375                 380

Glu Ala Asp Ala Met Ser Leu Asp Gly Gly Phe Val Tyr Ile Ala Gly
385                 390                 395                 400

Lys Cys Gly Leu Val Pro Val Leu Ala Glu Asn Tyr Asn Lys Ser Asp
                405                 410                 415

Asn Cys Glu Asp Thr Pro Glu Ala Gly Tyr Phe Ala Val Ala Val Val

420                    425                    430

Lys Lys Ser Ala Ser Asp Leu Thr Trp Asp Asn Leu Lys Gly Lys Lys
        435                    440                    445

Ser Cys His Thr Ala Val Gly Arg Thr Ala Gly Trp Asn Ile Pro Met
    450                    455                    460

Gly Leu Leu Tyr Asn Lys Ile Asn His Cys Arg Phe Asp Glu Phe Phe
465                    470                    475                    480

Ser Glu Gly Cys Ala Pro Gly Ser Lys Lys Asp Ser Ser Leu Cys Lys
                485                    490                    495

Leu Cys Met Gly Ser Gly Leu Asn Leu Cys Glu Pro Asn Asn Lys Glu
            500                    505                    510

Gly Tyr Tyr Gly Tyr Thr Gly Ala Phe Arg Cys Leu Val Glu Lys Gly
        515                    520                    525

Asp Val Ala Phe Val Lys His Gln Thr Val Pro Gln Asn Thr Gly Gly
    530                    535                    540

Lys Asn Pro Asp Pro Trp Ala Lys Asn Leu Asn Glu Lys Asp Tyr Glu
545                    550                    555                    560

Leu Leu Cys Leu Asp Gly Thr Arg Lys Pro Val Glu Glu Tyr Ala Asn
                565                    570                    575

Cys His Leu Ala Arg Ala Pro Asn His Ala Val Val Thr Arg Lys Asp
            580                    585                    590

Lys Glu Ala Cys Val His Lys Ile Leu Arg Gln Gln Gln His Leu Phe
        595                    600                    605

Gly Ser Asn Val Thr Asp Cys Ser Gly Asn Phe Cys Leu Phe Arg Ser
    610                    615                    620

Glu Thr Lys Asp Leu Leu Phe Arg Asp Asp Thr Val Cys Leu Ala Lys
625                    630                    635                    640

Leu His Asp Arg Asn Thr Tyr Glu Lys Tyr Leu Gly Glu Glu Tyr Val
                645                    650                    655

Lys Ala Val Gly Asn Leu Arg Lys Cys Ser Thr Ser Ser Leu Leu Glu
            660                    665                    670

Ala Cys Thr Phe Arg Arg Pro
        675


<210>    3
<211>    5
<212>    PRT
<213>    Artificial Sequence

<220>
<223>    Linker GS-1


<400>    3
Gly Gly Gly Gly Ser
1               5


<210>    4
<211>    15
<212>    PRT

<213>    Artificial Sequence

<220>
<223>    Linker GS-3


<400>    4
Gly Gly Gly Gly Ser Gly Gly Gly Gly Ser Gly Gly Gly Gly Ser
1               5                   10                  15


<210>    5
<211>    25
<212>    PRT
<213>    Artificial Sequence

<220>
<223>    Linker GS-5


<400>    5
Gly Gly Gly Gly Ser Gly Gly Gly Gly Ser Gly Gly Gly Gly Ser Gly
1               5                   10                  15

Gly Gly Gly Ser Gly Gly Gly Gly Ser
            20                  25


<210>    6
<211>    35
<212>    PRT
<213>    Artificial Sequence

<220>
<223>    Linker GS-7


<400>    6
Gly Gly Gly Gly Ser Gly Gly Gly Gly Ser Gly Gly Gly Gly Ser Gly
1               5                   10                  15

Gly Gly Gly Ser Gly Gly Gly Gly Ser Gly Gly Gly Gly Ser Gly Gly
            20                  25                  30

Gly Gly Ser
        35


<210>    7
<211>    45
<212>    PRT
<213>    Artificial Sequence

<220>
<223>    Linker GS-9


<400>    7
Gly Gly Gly Gly Ser Gly Gly Gly Gly Ser Gly Gly Gly Gly Ser Gly
1               5                   10                  15

Gly Gly Gly Ser Gly Gly Gly Gly Ser Gly Gly Gly Gly Ser Gly Gly
            20                  25                  30

Gly Gly Ser Gly Gly Gly Gly Ser Gly Gly Gly Gly Ser
        35                  40                  45

<210>    8
<211>    55
<212>    PRT
<213>    Artificial Sequence

<220>
<223>    Linker GS-11


<400>    8
Gly Gly Gly Gly Ser Gly Gly Gly Gly Ser Gly Gly Gly Gly Ser Gly
1               5                   10                  15

Gly Gly Gly Ser Gly Gly Gly Gly Ser Gly Gly Gly Gly Ser Gly Gly
            20                  25                  30

Gly Gly Ser Gly Gly Gly Gly Ser Gly Gly Gly Gly Ser Gly Gly Gly
        35                  40                  45

Gly Ser Gly Gly Gly Gly Ser
    50                  55


<210>    9
<211>    65
<212>    PRT
<213>    Artificial Sequence

<220>
<223>    Linker GS-13


<400>    9
Gly Gly Gly Gly Ser Gly Gly Gly Gly Ser Gly Gly Gly Gly Ser Gly
1               5                   10                  15

Gly Gly Gly Ser Gly Gly Gly Gly Ser Gly Gly Gly Gly Ser Gly Gly
            20                  25                  30

Gly Gly Ser Gly Gly Gly Gly Ser Gly Gly Gly Gly Ser Gly Gly Gly
        35                  40                  45

Gly Ser Gly Gly Gly Gly Ser Gly Gly Gly Gly Ser Gly Gly Gly Gly
    50                  55                  60

Ser
65


<210>    10
<211>    75
<212>    PRT
<213>    Artificial Sequence

<220>
<223>    Linker GS-15


<400>    10
Gly Gly Gly Gly Ser Gly Gly Gly Gly Ser Gly Gly Gly Gly Ser Gly
1               5                   10                  15

Gly Gly Gly Ser Gly Gly Gly Gly Ser Gly Gly Gly Gly Ser Gly Gly
            20                  25                  30

Gly Gly Ser Gly Gly Gly Gly Ser Gly Gly Gly Gly Ser Gly Gly Gly
        35                  40                  45

Gly Ser Gly Gly Gly Gly Ser Gly Gly Gly Gly Ser Gly Gly Gly Gly
    50                  55                  60

Ser Gly Gly Gly Gly Ser Gly Gly Gly Gly Ser
65                  70                  75

<210>    11
<211>    23
<212>    PRT
<213>    Artificial Sequence

<220>
<223>    Linker Helix

<400>    11
Gly Ala Glu Ala Ala Ala Lys Glu Ala Ala Ala Lys Glu Ala Ala Ala
1               5                   10                  15

Lys Glu Ala Ala Ala Lys Ala
            20

<210>    12
<211>    15
<212>    PRT
<213>    Artificial Sequence

<220>
<223>    Linker GS1-T

<400>    12
Gly Gly Gly Gly Ser Leu Val Pro Arg Gly Ser Gly Gly Gly Ser
1               5                   10                  15

<210>    13
<211>    3378
<212>    DNA
<213>    Artificial Sequence

<220>
<223>    gene sequence encoding FVII-Tf protein

<400>    13
atggtctccc aggccctcag gctcctctgc cttctgcttg ggcttcaggg ctgcctggct        60

gcagtcttcg taacccagga ggaagcccac ggcgtcctgc accggcgccg gcgcgccaac       120

gcgttcctgg aggagctgcg gccgggctcc ctggagaggg agtgcaagga ggagcagtgc       180

tccttcgagg aggcccggga gatcttcaag gacgcggaga ggacgaagct gttctggatt       240

tcttacagtg atggggacca gtgtgcctca agtccatgcc agaatggggg ctcctgcaag       300

gaccagctcc agtcctatat ctgcttctgc ctccctgcct tcgagggccg gaactgtgag       360

acgcacaagg atgaccagct gatctgtgtg aacgagaacg gcggctgtga gcagtactgc       420

agtgaccaca cgggcaccaa gcgctcctgt cggtgccacg aggggtactc tctgctggca       480

gacggggtgt cctgcacacc cacagttgaa tatccatgtg gaaaaatacc tattctagaa       540

aaaagaaatg ccagcaaacc ccaaggccga attgtggggg gcaaggtgtg ccccaaaggg       600

gagtgtccat ggcaggtcct gttgttggtg aatggagctc agttgtgtgg ggggaccctg 660
atcaacacca tctgggtggt ctccgcggcc cactgtttcg acaaaatcaa gaactggagg 720
aacctgatcg cggtgctggg cgagcacgac ctcagcgagc acgacgggga tgagcagagc 780
cggcgggtgg cgcaggtcat catccccagc acgtacgtcc cgggcaccac caaccacgac 840
atcgcgctgc tccgcctgca ccagcccgtg gtcctcactg accatgtggt gcccctctgc 900
ctgcccgaac ggacgttctc tgagaggacg ctggccttcg tgcgcttctc attggtcagc 960
ggctggggcc agctgctgga ccgtggcgcc acggccctgg agctcatggt cctcaacgtg 1020
ccccggctga tgacccagga ctgcctgcag cagtcacgga aggtgggaga ctccccaaat 1080
atcacggagt acatgttctg tgccggctac tcggatggca gcaaggactc ctgcaagggg 1140
gacagtggag gcccacatgc cacccactac cggggcacgt ggtacctgac gggcatcgtc 1200
agctggggcc agggctgcgc aaccgtgggc cactttgggg tgtacaccag ggtctcccag 1260
tacatcgagt ggctgcaaaa gctcatgcgc tcagagccac gcccaggagt cctcctgcga 1320
gccccatttc ccaccggtgt ccctgataaa actgtgagat ggtgtgcagt gtcggagcat 1380
gaggccacta agtgccagag tttccgcgac catatgaaaa gcgtcattcc atccgatggt 1440
cccagtgttg cttgtgtgaa gaaagcctcc taccttgatt gcatcagggc cattgcggca 1500
aacgaagcgg atgctgtgac actggatgca ggtttggtgt atgatgctta cctggctccc 1560
aataacctga agcctgtggt ggcagagttc tatgggtcaa aagaggatcc acagactttc 1620
tattatgctg ttgctgtggt gaagaaggat agtggcttcc agatgaacca gcttcgaggc 1680
aagaagtcct gccacacggg tctaggcagg tccgctgggt ggaacatccc cataggctta 1740
ctttactgtg acttacctga gccacgtaaa cctcttgaga aagcagtggc caatttcttc 1800
tcgggcagct gtgccccttg tgcggatggg acggacttcc cccagctgtg tcaactgtgt 1860
ccagggtgtg gctgctccac ccttaaccaa tacttcggct actcaggagc cttcaagtgt 1920
ctgaaggatg gtgctgggga tgtggccttt gtcaagcact cgactatatt tgagaacttg 1980
gcaaacaagg ctgacaggga ccagtatgag ctgctttgcc tggacaacac ccggaagccg 2040
gtagatgaat acaaggactg ccacttggcc caggtccctt ctcataccgt cgtggcccga 2100
agtatgggcg gcaaggagga cttgatctgg gagcttctca accaggccca ggaacatttt 2160
ggcaaagaca aatcaaaaga attccaacta ttcagctctc ctcatgggaa ggacctgctg 2220
tttaaggact ctgcccacgg gtttttaaaa gtccccccca ggatggatgc caagatgtac 2280
ctgggctatg agtatgtcac tgccatccgg aatctacggg aaggcacatg cccagaagcc 2340
ccaacagatg aatgcaagcc tgtgaagtgg tgtgcgctga gccaccacga gaggctcaag 2400
tgtgatgagt ggagtgttaa cagtgtaggg aaaatagagt gtgtatcagc agagaccacc 2460
gaagactgca tcgccaagat catgaatgga gaagctgatg ccatgagctt ggatggaggg 2520
tttgtctaca tagcgggcaa gtgtggtctg gtgcctgtct tggcagaaaa ctacaataag 2580
agcgataatt gtgaggatac accagaggca gggtattttg ctgtagcagt ggtgaagaaa 2640

tcagcttctg acctcacctg ggacaatctg aaaggcaaga agtcctgcca tacggcagtt 2700

ggcagaaccg ctggctggaa catccccatg ggcctgctct acaataagat caaccactgc 2760

agatttgatg aatttttcag tgaaggttgt gcccctgggt ctaagaaaga ctccagtctc 2820

tgtaagctgt gtatgggctc aggcctaaac ctgtgtgaac ccaacaacaa agagggatac 2880

tacggctaca caggcgcttt caggtgtctg gttgagaagg gagatgtggc ctttgtgaaa 2940

caccagactg tcccacagaa cactggggga aaaaaccctg atccatgggc taagaatctg 3000

aatgaaaaag actatgagtt gctgtgcctt gatggtacca ggaaacctgt ggaggagtat 3060

gcgaactgcc acctggccag agccccgaat cacgctgtgg tcacacggaa agataaggaa 3120

gcttgcgtcc acaagatatt acgtcaacag cagcacctat ttggaagcaa cgtaactgac 3180

tgctcgggca actttgtttt gttccggtcg gaaaccaagg accttctgtt cagagatgac 3240

acagtatgtt tggccaaact tcatgacaga aacacatatg aaaaatactt aggagaagaa 3300

tatgtcaagg ctgttggtaa cctgagaaaa tgctccacct catcactcct ggaagcctgc 3360

actttccgta gaccttaa 3378

<210> 14
<211> 3387
<212> DNA
<213> Artificial Sequence

<220>
<223> gene sequence encoding FVII-GS1-Tf protein

<400> 14
atggtctccc aggccctcag gctcctctgc cttctgcttg ggcttcaggg ctgcctggct 60

gcagtcttcg taacccagga ggaagcccac ggcgtcctgc accggcgccg gcgcgccaac 120

gcgttcctgg aggagctgcg gccgggctcc ctggagaggg agtgcaagga ggagcagtgc 180

tccttcgagg aggcccggga gatcttcaag gacgcggaga ggacgaagct gttctggatt 240

tcttacagtg atggggacca gtgtgcctca agtccatgcc agaatggggg ctcctgcaag 300

gaccagctcc agtcctatat ctgcttctgc ctccctgcct tcgagggccg gaactgtgag 360

acgcacaagg atgaccagct gatctgtgtg aacgagaacg gcggctgtga gcagtactgc 420

agtgaccaca cgggcaccaa gcgctcctgt cggtgccacg aggggtactc tctgctggca 480

gacggggtgt cctgcacacc cacagttgaa tatccatgtg gaaaaatacc tattctagaa 540

aaaagaaatg ccagcaaacc ccaaggccga attgtggggg gcaaggtgtg ccccaaaggg 600

gagtgtccat ggcaggtcct gttgttggtg aatggagctc agttgtgtgg ggggaccctg 660

atcaacacca tctgggtggt ctccgcggcc cactgtttcg acaaaatcaa gaactggagg 720

aacctgatcg cggtgctggg cgagcacgac ctcagcgagc acgacgggga tgagcagagc 780

cggcgggtgg cgcaggtcat catccccagc acgtacgtcc cgggcaccac caaccacgac 840

atcgcgctgc tccgcctgca ccagcccgtg gtcctcactg accatgtggt gcccctctgc 900

ctgcccgaac ggacgttctc tgagaggacg ctggccttcg tgcgcttctc attggtcagc 960

ggctggggcc agctgctgga ccgtggcgcc acggccctgg agctcatggt cctcaacgtg 1020
ccccggctga tgacccagga ctgcctgcag cagtcacgga aggtgggaga ctccccaaat 1080
atcacggagt acatgttctg tgccggctac tcggatggca gcaaggactc ctgcaagggg 1140
gacagtggag gcccacatgc cacccactac cggggcacgt ggtacctgac gggcatcgtc 1200
agctggggcc agggctgcgc aaccgtgggc cactttgggg tgtacaccag ggtctcccag 1260
tacatcgagt ggctgcaaaa gctcatgcgc tcagagccac gcccaggagt cctcctgcga 1320
gccccatttc ccggtggagg cggatccgtc cctgataaaa ctgtgagatg gtgtgcagtg 1380
tcggagcatg aggccactaa gtgccagagt ttccgcgacc atatgaaaag cgtcattcca 1440
tccgatggtc ccagtgttgc ttgtgtgaag aaagcctcct accttgattg catcagggcc 1500
attgcggcaa acgaagcgga tgctgtgaca ctggatgcag gtttggtgta tgatgcttac 1560
ctggctccca ataacctgaa gcctgtggtg gcagagttct atgggtcaaa agaggatcca 1620
cagactttct attatgctgt tgctgtggtg aagaaggata gtggcttcca gatgaaccag 1680
cttcgaggca agaagtcctg ccacacgggt ctaggcaggt ccgctgggtg gaacatcccc 1740
ataggcttac tttactgtga cttacctgag ccacgtaaac ctcttgagaa agcagtggcc 1800
aatttcttct cgggcagctg tgccccttgt gcggatggga cggacttccc ccagctgtgt 1860
caactgtgtc cagggtgtgg ctgctccacc cttaaccaat acttcggcta ctcaggagcc 1920
ttcaagtgtc tgaaggatgg tgctggggat gtggcctttg tcaagcactc gactatattt 1980
gagaacttgg caaacaaggc tgacagggac cagtatgagc tgctttgcct ggacaacacc 2040
cggaagccgg tagatgaata caaggactgc cacttggccc aggtcccttc tcataccgtc 2100
gtggcccgaa gtatgggcgg caaggaggac ttgatctggg agcttctcaa ccaggcccag 2160
gaacattttg gcaaagacaa atcaaaagaa ttccaactat tcagctctcc tcatgggaag 2220
gacctgctgt ttaaggactc tgcccacggg tttttaaaag tcccccccag gatggatgcc 2280
aagatgtacc tgggctatga gtatgtcact gccatccgga atctacggga aggcacatgc 2340
ccagaagccc caacagatga atgcaagcct gtgaagtggt gtgcgctgag ccaccacgag 2400
aggctcaagt gtgatgagtg gagtgttaac agtgtaggga aaatagagtg tgtatcagca 2460
gagaccaccg aagactgcat cgccaagatc atgaatggag aagctgatgc catgagcttg 2520
gatggagggt ttgtctacat agcgggcaag tgtggtctgg tgcctgtctt ggcagaaaac 2580
tacaataaga gcgataattg tgaggataca ccagaggcag ggtattttgc tgtagcagtg 2640
gtgaagaaat cagcttctga cctcacctgg gacaatctga aaggcaagaa gtcctgccat 2700
acggcagttg gcagaaccgc tggctggaac atccccatgg gcctgctcta caataagatc 2760
aaccactgca gatttgatga atttttcagt gaaggttgtg cccctgggtc taagaaagac 2820
tccagtctct gtaagctgtg tatgggctca ggcctaaacc tgtgtgaacc caacaacaaa 2880
gagggatact acggctacac aggcgctttc aggtgtctgg ttgagaaggg agatgtggcc 2940
tttgtgaaac accagactgt cccacagaac actgggggaa aaaaccctga tccatgggct 3000

aagaatctga atgaaaaaga ctatgagttg ctgtgccttg atggtaccag gaaacctgtg 3060

gaggagtatg cgaactgcca cctggccaga gccccgaatc acgctgtggt cacacggaaa 3120

gataaggaag cttgcgtcca caagatatta cgtcaacagc agcacctatt tggaagcaac 3180

gtaactgact gctcgggcaa cttttgtttg ttccggtcgg aaaccaagga ccttctgttc 3240

agagatgaca cagtatgttt ggccaaactt catgacagaa acacatatga aaaatactta 3300

ggagaagaat atgtcaaggc tgttggtaac ctgagaaaat gctccacctc atcactcctg 3360

gaagcctgca ctttccgtag accttaa 3387

<210> 15
<211> 3426
<212> DNA
<213> Artificial Sequence

<220>
<223> gene sequence encoding FVII-GS3-Tf protein

<400> 15
atggtctccc aggccctcag gctcctctgc cttctgcttg ggcttcaggg ctgcctggct 60

gcagtcttcg taacccagga ggaagcccac ggcgtcctgc accggcgccg gcgcgccaac 120

gcgttcctgg aggagctgcg gccgggctcc ctggagaggg agtgcaagga ggagcagtgc 180

tccttcgagg aggcccggga gatcttcaag gacgcggaga ggacgaagct gttctggatt 240

tcttacagtg atggggacca gtgtgcctca agtccatgcc agaatggggg ctcctgcaag 300

gaccagctcc agtcctatat ctgcttctgc ctccctgcct tcgagggccg gaactgtgag 360

acgcacaagg atgaccagct gatctgtgtg aacgagaacg gcggctgtga gcagtactgc 420

agtgaccaca cgggcaccaa gcgctcctgt cggtgccacg aggggtactc tctgctggca 480

gacggggtgt cctgcacacc cacagttgaa tatccatgtg gaaaaatacc tattctagaa 540

aaaagaaatg ccagcaaacc ccaaggccga attgtggggg gcaaggtgtg ccccaaaggg 600

gagtgtccat ggcaggtcct gttgttggtg aatggagctc agttgtgtgg ggggaccctg 660

atcaacacca tctgggtggt ctccgcggcc cactgtttcg acaaaatcaa gaactggagg 720

aacctgatcg cggtgctggg cgagcacgac ctcagcgagc acgacgggga tgagcagagc 780

cggcgggtgg cgcaggtcat catccccagc acgtacgtcc cgggcaccac caaccacgac 840

atcgcgctgc tccgcctgca ccagcccgtg gtcctcactg accatgtggt gcccctctgc 900

ctgcccgaac ggacgttctc tgagaggacg ctggccttcg tgcgcttctc attggtcagc 960

ggctggggcc agctgctgga ccgtggcgcc acggccctgg agctcatggt cctcaacgtg 1020

ccccggctga tgacccagga ctgcctgcag cagtcacgga aggtgggaga ctccccaaat 1080

atcacggagt acatgttctg tgccggctac tcggatggca gcaaggactc ctgcaagggg 1140

gacagtggag gcccacatgc cacccactac cggggcacgt ggtacctgac gggcatcgtc 1200

agctggggcc agggctgcgc aaccgtgggc cactttgggg tgtacaccag ggtctcccag 1260

tacatcgagt ggctgcaaaa gctcatgcgc tcagagccac gcccaggagt cctcctgcga 1320
gccccatttc ccaccggtgg aggcggttca ggcggaggtg gctctggcgg tggcggatcc 1380
accggtgtcc ctgataaaac tgtgagatgg tgtgcagtgt cggagcatga ggccactaag 1440
tgccagagtt tccgcgacca tatgaaaagc gtcattccat ccgatggtcc cagtgttgct 1500
tgtgtgaaga aagcctccta ccttgattgc atcagggcca ttgcggcaaa cgaagcggat 1560
gctgtgacac tggatgcagg tttggtgtat gatgcttacc tggctcccaa taacctgaag 1620
cctgtggtgg cagagttcta tgggtcaaaa gaggatccac agactttcta ttatgctgtt 1680
gctgtggtga agaaggatag tggcttccag atgaaccagc ttcgaggcaa gaagtcctgc 1740
cacacgggtc taggcaggtc cgctgggtgg aacatcccca taggcttact ttactgtgac 1800
ttacctgagc cacgtaaacc tcttgagaaa gcagtggcca atttcttctc gggcagctgt 1860
gccccttgtg cggatgggac ggacttcccc cagctgtgtc aactgtgtcc agggtgtggc 1920
tgctccaccc ttaaccaata cttcggctac tcaggagcct tcaagtgtct gaaggatggt 1980
gctggggatg tggcctttgt caagcactcg actatatttg agaacttggc aaacaaggct 2040
gacagggacc agtatgagct gctttgcctg gacaacaccc ggaagccggt agatgaatac 2100
aaggactgcc acttggccca ggtcccttct cataccgtcg tggcccgaag tatgggcggc 2160
aaggaggact tgatctggga gcttctcaac caggcccagg aacattttgg caaagacaaa 2220
tcaaaagaat tccaactatt cagctctcct catgggaagg acctgctgtt taaggactct 2280
gcccacgggt ttttaaaagt cccccccagg atggatgcca agatgtacct gggctatgag 2340
tatgtcactg ccatccggaa tctacgggaa ggcacatgcc cagaagcccc aacagatgaa 2400
tgcaagcctg tgaagtggtg tgcgctgagc caccacgaga ggctcaagtg tgatgagtgg 2460
agtgttaaca gtgtagggaa aatagagtgt gtatcagcag agaccaccga agactgcatc 2520
gccaagatca tgaatggaga agctgatgcc atgagcttgg atggagggtt tgtctacata 2580
gcgggcaagt gtggtctggt gcctgtcttg gcagaaaact acaataagag cgataattgt 2640
gaggatacac cagaggcagg gtattttgct gtagcagtgg tgaagaaatc agcttctgac 2700
ctcacctggg acaatctgaa aggcaagaag tcctgccata cggcagttgg cagaaccgct 2760
ggctggaaca tccccatggg cctgctctac aataagatca accactgcag atttgatgaa 2820
tttttcagtg aaggttgtgc ccctgggtct aagaaagact ccagtctctg taagctgtgt 2880
atgggctcag gcctaaacct gtgtgaaccc aacaacaaag agggatacta cggctacaca 2940
ggcgctttca ggtgtctggt tgagaaggga gatgtggcct ttgtgaaaca ccagactgtc 3000
ccacagaaca ctgggggaaa aaaccctgat ccatgggcta agaatctgaa tgaaaaagac 3060
tatgagttgc tgtgccttga tggtaccagg aaacctgtgg aggagtatgc gaactgccac 3120
ctggccagag ccccgaatca cgctgtggtc acacggaaag ataaggaagc ttgcgtccac 3180
aagatattac gtcaacagca gcacctattt ggaagcaacg taactgactg ctcgggcaac 3240
ttttgtttgt tccggtcgga aaccaaggac cttctgttca gagatgacac agtatgtttg 3300

gccaaacttc atgacagaaa cacatatgaa aaatacttag gagaagaata tgtcaaggct 3360

gttggtaacc tgagaaaatg ctccacctca tcactcctgg aagcctgcac tttccgtaga 3420

ccttaa 3426

<210> 16
<211> 3456
<212> DNA
<213> Artificial Sequence

<220>
<223> gene sequence encoding FVII-GS5-Tf protein

<400> 16
atggtctccc aggccctcag gctcctctgc cttctgcttg ggcttcaggg ctgcctggct 60

gcagtcttcg taacccagga ggaagcccac ggcgtcctgc accggcgccg gcgcgccaac 120

gcgttcctgg aggagctgcg gccgggctcc ctggagaggg agtgcaagga ggagcagtgc 180

tccttcgagg aggcccggga gatcttcaag gacgcggaga ggacgaagct gttctggatt 240

tcttacagtg atggggacca gtgtgcctca agtccatgcc agaatggggg ctcctgcaag 300

gaccagctcc agtcctatat ctgcttctgc ctccctgcct tcgagggccg gaactgtgag 360

acgcacaagg atgaccagct gatctgtgtg aacgagaacg gcggctgtga gcagtactgc 420

agtgaccaca cgggcaccaa gcgctcctgt cggtgccacg aggggtactc tctgctggca 480

gacggggtgt cctgcacacc cacagttgaa tatccatgtg gaaaaatacc tattctagaa 540

aaaagaaatg ccagcaaacc ccaaggccga attgtggggg gcaaggtgtg ccccaaaggg 600

gagtgtccat ggcaggtcct gttgttggtg aatggagctc agttgtgtgg ggggaccctg 660

atcaacacca tctgggtggt ctccgcggcc cactgtttcg acaaaatcaa gaactggagg 720

aacctgatcg cggtgctggg cgagcacgac ctcagcgagc acgacgggga tgagcagagc 780

cggcgggtgg cgcaggtcat catccccagc acgtacgtcc cgggcaccac caaccacgac 840

atcgcgctgc tccgcctgca ccagcccgtg gtcctcactg accatgtggt gcccctctgc 900

ctgcccgaac ggacgttctc tgagaggacg ctggccttcg tgcgcttctc attggtcagc 960

ggctggggcc agctgctgga ccgtggcgcc acggccctgg agctcatggt cctcaacgtg 1020

ccccggctga tgacccagga ctgcctgcag cagtcacgga aggtgggaga ctccccaaat 1080

atcacggagt acatgttctg tgccggctac tcggatggca gcaaggactc ctgcaagggg 1140

gacagtggag gcccacatgc cacccactac cggggcacgt ggtacctgac gggcatcgtc 1200

agctggggcc agggctgcgc aaccgtgggc cactttgggg tgtacaccag ggtctcccag 1260

tacatcgagt ggctgcaaaa gctcatgcgc tcagagccac gcccaggagt cctcctgcga 1320

gccccatttc ccaccggtgg aggcggttca ggcggaggtg gctctggcgg tggcggatct 1380

ggcggaggtg gctctggcgg tggcggatcc accggtgtcc ctgataaaac tgtgagatgg 1440

tgtgcagtgt cggagcatga ggccactaag tgccagagtt tccgcgacca tatgaaaagc 1500

gtcattccat ccgatggtcc cagtgttgct tgtgtgaaga aagcctccta ccttgattgc 1560

atcagggcca ttgcggcaaa cgaagcggat gctgtgacac tggatgcagg tttggtgtat 1620
gatgcttacc tggctcccaa taacctgaag cctgtggtgg cagagttcta tgggtcaaaa 1680
gaggatccac agactttcta ttatgctgtt gctgtggtga agaaggatag tggcttccag 1740
atgaaccagc ttcgaggcaa gaagtcctgc cacacgggtc taggcaggtc cgctgggtgg 1800
aacatcccca taggcttact ttactgtgac ttacctgagc cacgtaaacc tcttgagaaa 1860
gcagtggcca atttcttctc gggcagctgt gccccttgtg cggatgggac ggacttcccc 1920
cagctgtgtc aactgtgtcc agggtgtggc tgctccaccc ttaaccaata cttcggctac 1980
tcaggagcct tcaagtgtct gaaggatggt gctggggatg tggcctttgt caagcactcg 2040
actatatttg agaacttggc aaacaaggct gacagggacc agtatgagct gctttgcctg 2100
gacaacaccc ggaagccggt agatgaatac aaggactgcc acttggccca ggtcccttct 2160
cataccgtcg tggcccgaag tatgggcggc aaggaggact tgatctggga gcttctcaac 2220
caggcccagg aacatttggg caaagacaaa tcaaaagaat tccaactatt cagctctcct 2280
catgggaagg acctgctgtt taaggactct gcccacgggt ttttaaaagt cccccccagg 2340
atggatgcca agatgtacct gggctatgag tatgtcactg ccatccggaa tctacgggaa 2400
ggcacatgcc cagaagcccc aacagatgaa tgcaagcctg tgaagtggtg tgcgctgagc 2460
caccacgaga ggctcaagtg tgatgagtgg agtgttaaca gtgtagggaa aatagagtgt 2520
gtatcagcag agaccaccga agactgcatc gccaagatca tgaatggaga agctgatgcc 2580
atgagcttgg atggagggtt tgtctacata gcgggcaagt gtggtctggt gcctgtcttg 2640
gcagaaaact acaataagag cgataattgt gaggatacac cagaggcagg gtattttgct 2700
gtagcagtgg tgaagaaatc agcttctgac ctcacctggg acaatctgaa aggcaagaag 2760
tcctgccata cggcagttgg cagaaccgct ggctggaaca tccccatggg cctgctctac 2820
aataagatca accactgcag atttgatgaa tttttcagtg aaggttgtgc ccctgggtct 2880
aagaaagact ccagtctctg taagctgtgt atgggctcag gcctaaacct gtgtgaaccc 2940
aacaacaaag agggatacta cggctacaca ggcgctttca ggtgtctggt tgagaaggga 3000
gatgtggcct ttgtgaaaca ccagactgtc ccacagaaca ctgggggaaa aaaccctgat 3060
ccatgggcta agaatctgaa tgaaaaagac tatgagttgc tgtgccttga tggtaccagg 3120
aaacctgtgg aggagtatgc gaactgccac ctggccagag ccccgaatca cgctgtggtc 3180
acacggaaag ataaggaagc ttgcgtccac aagatattac gtcaacagca gcacctattt 3240
ggaagcaacg taactgactg ctcgggcaac ttttgtttgt tccggtcgga aaccaaggac 3300
cttctgttca gagatgacac agtatgtttg gccaaacttc atgacagaaa cacatatgaa 3360
aaatacttag gagaagaata tgtcaaggct gttggtaacc tgagaaaatg ctccacctca 3420
tcactcctgg aagcctgcac tttccgtaga ccttaa 3456

<210> 17
<211> 3486

<212>    DNA
<213>    Artificial Sequence

<220>
<223>    gene sequence encoding FVII-GS7-Tf protein

<400>    17
atggtctccc aggccctcag gctcctctgc cttctgcttg ggcttcaggg ctgcctggct      60
gcagtcttcg taacccagga ggaagcccac ggcgtcctgc accggcgccg gcgcgccaac     120
gcgttcctgg aggagctgcg gccgggctcc ctggagaggg agtgcaagga ggagcagtgc     180
tccttcgagg aggcccggga gatcttcaag gacgcggaga ggacgaagct gttctggatt     240
tcttacagtg atggggacca gtgtgcctca agtccatgcc agaatggggg ctcctgcaag     300
gaccagctcc agtcctatat ctgcttctgc ctccctgcct tcgagggccg gaactgtgag     360
acgcacaagg atgaccagct gatctgtgtg aacgagaacg gcggctgtga gcagtactgc     420
agtgaccaca cgggcaccaa gcgctcctgt cggtgccacg aggggtactc tctgctggca     480
gacggggtgt cctgcacacc cacagttgaa tatccatgtg gaaaaatacc tattctagaa     540
aaaagaaatg ccagcaaacc ccaaggccga attgtggggg gcaaggtgtg ccccaaaggg     600
gagtgtccat ggcaggtcct gttgttggtg aatggagctc agttgtgtgg ggggaccctg     660
atcaacacca tctgggtggt ctccgcggcc cactgtttcg acaaaatcaa gaactggagg     720
aacctgatcg cggtgctggg cgagcacgac ctcagcgagc acgacgggga tgagcagagc     780
cggcgggtgg cgcaggtcat catccccagc acgtacgtcc cgggcaccac caaccacgac     840
atcgcgctgc tccgcctgca ccagcccgtg gtcctcactg accatgtggt gcccctctgc     900
ctgcccgaac ggacgttctc tgagaggacg ctggccttcg tgcgcttctc attggtcagc     960
ggctggggcc agctgctgga ccgtggcgcc acggccctgg agctcatggt cctcaacgtg    1020
ccccggctga tgacccagga ctgcctgcag cagtcacgga aggtgggaga ctccccaaat    1080
atcacggagt acatgttctg tgccggctac tcggatggca gcaaggactc ctgcaagggg    1140
gacagtggag gcccacatgc cacccactac cggggcacgt ggtacctgac gggcatcgtc    1200
agctggggcc agggctgcgc aaccgtgggc cactttgggg tgtacaccag ggtctcccag    1260
tacatcgagt ggctgcaaaa gctcatgcgc tcagagccac gcccaggagt cctcctgcga    1320
gccccatttc ccaccggtgg aggcggttca ggcggaggtg gctctggcgg tggcggatct    1380
ggcggaggtg gctctggcgg tggcggatct ggcggaggtg gctctggcgg tggcggatcc    1440
accggtgtcc ctgataaaac tgtgagatgg tgtgcagtgt cggagcatga ggccactaag    1500
tgccagagtt tccgcgacca tatgaaaagc gtcattccat ccgatggtcc cagtgttgct    1560
tgtgtgaaga aagcctccta ccttgattgc atcagggcca ttgcggcaaa cgaagcggat    1620
gctgtgacac tggatgcagg tttggtgtat gatgcttacc tggctcccaa taacctgaag    1680
cctgtggtgg cagagttcta tgggtcaaaa gaggatccac agactttcta ttatgctgtt    1740
gctgtggtga agaaggatag tggcttccag atgaaccagc ttcgaggcaa gaagtcctgc    1800

cacacgggtc taggcaggtc cgctgggtgg aacatcccca taggcttact ttactgtgac 1860

ttacctgagc cacgtaaacc tcttgagaaa gcagtggcca atttcttctc gggcagctgt 1920

gccccttgtg cggatgggac ggacttcccc cagctgtgtc aactgtgtcc agggtgtggc 1980

tgctccaccc ttaaccaata cttcggctac tcaggagcct tcaagtgtct gaaggatggt 2040

gctggggatg tggcctttgt caagcactcg actatatttg agaacttggc aaacaaggct 2100

gacagggacc agtatgagct gctttgcctg gacaacaccc ggaagccggt agatgaatac 2160

aaggactgcc acttggccca ggtcccttct cataccgtcg tggcccgaag tatgggcggc 2220

aaggaggact tgatctggga gcttctcaac caggcccagg aacattttgg caaagacaaa 2280

tcaaaagaat tccaactatt cagctctcct catgggaagg acctgctgtt taaggactct 2340

gcccacgggt ttttaaaagt cccccccagg atggatgcca agatgtacct gggctatgag 2400

tatgtcactg ccatccggaa tctacgggaa ggcacatgcc cagaagcccc aacagatgaa 2460

tgcaagcctg tgaagtggtg tgcgctgagc caccacgaga ggctcaagtg tgatgagtgg 2520

agtgttaaca gtgtagggaa aatagagtgt gtatcagcag agaccaccga agactgcatc 2580

gccaagatca tgaatggaga agctgatgcc atgagcttgg atggagggtt tgtctacata 2640

gcgggcaagt gtggtctggt gcctgtcttg gcagaaaact acaataagag cgataattgt 2700

gaggatacac cagaggcagg gtattttgct gtagcagtgg tgaagaaatc agcttctgac 2760

ctcacctggg acaatctgaa aggcaagaag tcctgccata cggcagttgg cagaaccgct 2820

ggctggaaca tccccatggg cctgctctac aataagatca accactgcag atttgatgaa 2880

tttttcagtg aaggttgtgc ccctgggtct aagaaagact ccagtctctg taagctgtgt 2940

atgggctcag gcctaaacct gtgtgaaccc aacaacaaag agggatacta cggctacaca 3000

ggcgctttca ggtgtctggt tgagaaggga gatgtggcct ttgtgaaaca ccagactgtc 3060

ccacagaaca ctgggggaaa aaaccctgat ccatgggcta agaatctgaa tgaaaaagac 3120

tatgagttgc tgtgccttga tggtaccagg aaacctgtgg aggagtatgc gaactgccac 3180

ctggccagag ccccgaatca cgctgtggtc acacggaaag ataaggaagc ttgcgtccac 3240

aagatattac gtcaacagca gcacctattt ggaagcaacg taactgactg ctcgggcaac 3300

ttttgtttgt tccggtcgga aaccaaggac cttctgttca gagatgacac agtatgtttg 3360

gccaaacttc atgacagaaa cacatatgaa aaatacttag gagaagaata tgtcaaggct 3420

gttggtaacc tgagaaaatg ctccacctca tcactcctgg aagcctgcac tttccgtaga 3480

ccttaa 3486

<210> 18
<211> 3516
<212> DNA
<213> Artificial Sequence

<220>
<223> gene sequence encoding FVII-GS9-Tf protein

<400> 18
atggtctccc aggccctcag gctcctctgc cttctgcttg ggcttcaggg ctgcctggct 60
gcagtcttcg taacccagga ggaagcccac ggcgtcctgc accggcgccg gcgcgccaac 120
gcgttcctgg aggagctgcg gccgggctcc ctggagaggg agtgcaagga ggagcagtgc 180
tccttcgagg aggcccggga gatcttcaag gacgcggaga ggacgaagct gttctggatt 240
tcttacagtg atggggacca gtgtgcctca agtccatgcc agaatggggg ctcctgcaag 300
gaccagctcc agtcctatat ctgcttctgc ctccctgcct tcgagggccg gaactgtgag 360
acgcacaagg atgaccagct gatctgtgtg aacgagaacg gcggctgtga gcagtactgc 420
agtgaccaca cgggcaccaa gcgctcctgt cggtgccacg aggggtactc tctgctggca 480
gacggggtgt cctgcacacc cacagttgaa tatccatgtg gaaaaatacc tattctagaa 540
aaaagaaatg ccagcaaacc ccaaggccga attgtggggg gcaaggtgtg ccccaaaggg 600
gagtgtccat ggcaggtcct gttgttggtg aatggagctc agttgtgtgg ggggaccctg 660
atcaacacca tctgggtggt ctccgcggcc cactgtttcg acaaaatcaa gaactggagg 720
aacctgatcg cggtgctggg cgagcacgac ctcagcgagc acgacgggga tgagcagagc 780
cggcgggtgg cgcaggtcat catccccagc acgtacgtcc cgggcaccac caaccacgac 840
atcgcgctgc tccgcctgca ccagcccgtg gtcctcactg accatgtggt gcccctctgc 900
ctgcccgaac ggacgttctc tgagaggacg ctggccttcg tgcgcttctc attggtcagc 960
ggctggggcc agctgctgga ccgtggcgcc acggccctgg agctcatggt cctcaacgtg 1020
ccccggctga tgacccagga ctgcctgcag cagtcacgga aggtgggaga ctccccaaat 1080
atcacggagt acatgttctg tgccggctac tcggatggca gcaaggactc ctgcaagggg 1140
gacagtggag gcccacatgc cacccactac cggggcacgt ggtacctgac gggcatcgtc 1200
agctggggcc agggctgcgc aaccgtgggc cactttgggg tgtacaccag ggtctcccag 1260
tacatcgagt ggctgcaaaa gctcatgcgc tcagagccac gcccaggagt cctcctgcga 1320
gccccatttc ccaccggtgg aggcggttca ggcggaggtg gctctggcgg tggcggatct 1380
ggcggaggtg gctctggcgg tggcggatct ggcggaggtg gctctggcgg tggcggatct 1440
ggcggaggtg gctctggcgg tggcggatcc accggtgtcc ctgataaaac tgtgagatgg 1500
tgtgcagtgt cggagcatga ggccactaag tgccagagtt tccgcgacca tatgaaaagc 1560
gtcattccat ccgatggtcc cagtgttgct tgtgtgaaga aagcctccta ccttgattgc 1620
atcagggcca ttgcggcaaa cgaagcggat gctgtgacac tggatgcagg tttggtgtat 1680
gatgcttacc tggctcccaa taacctgaag cctgtggtgg cagagttcta tgggtcaaaa 1740
gaggatccac agactttcta ttatgctgtt gctgtggtga agaaggatag tggcttccag 1800
atgaaccagc ttcgaggcaa gaagtcctgc cacacgggtc taggcaggtc cgctgggtgg 1860
aacatcccca taggcttact ttactgtgac ttacctgagc cacgtaaacc tcttgagaaa 1920
gcagtggcca atttcttctc gggcagctgt gccccttgtg cggatgggac ggacttcccc 1980
cagctgtgtc aactgtgtcc agggtgtggc tgctccaccc ttaaccaata cttcggctac 2040

tcaggagcct tcaagtgtct gaaggatggt gctggggatg tggcctttgt caagcactcg 2100

actatatttg agaacttggc aaacaaggct gacagggacc agtatgagct gctttgcctg 2160

gacaacaccc ggaagccggt agatgaatac aaggactgcc acttggccca ggtcccttct 2220

cataccgtcg tggcccgaag tatgggcggc aaggaggact tgatctggga gcttctcaac 2280

caggcccagg aacattttgg caaagacaaa tcaaaagaat tccaactatt cagctctcct 2340

catgggaagg acctgctgtt taaggactct gcccacgggt ttttaaaagt cccccccagg 2400

atggatgcca agatgtacct gggctatgag tatgtcactg ccatccggaa tctacgggaa 2460

ggcacatgcc cagaagcccc aacagatgaa tgcaagcctg tgaagtggtg tgcgctgagc 2520

caccacgaga ggctcaagtg tgatgagtgg agtgttaaca gtgtagggaa aatagagtgt 2580

gtatcagcag agaccaccga agactgcatc gccaagatca tgaatggaga agctgatgcc 2640

atgagcttgg atggagggtt tgtctacata gcgggcaagt gtggtctggt gcctgtcttg 2700

gcagaaaact acaataagag cgataattgt gaggatacac cagaggcagg gtattttgct 2760

gtagcagtgg tgaagaaatc agcttctgac ctcacctggg acaatctgaa aggcaagaag 2820

tcctgccata cggcagttgg cagaaccgct ggctggaaca tccccatggg cctgctctac 2880

aataagatca accactgcag atttgatgaa tttttcagtg aaggttgtgc ccctgggtct 2940

aagaaagact ccagtctctg taagctgtgt atgggctcag gcctaaacct gtgtgaaccc 3000

aacaacaaag agggatacta cggctacaca ggcgctttca ggtgtctggt tgagaaggga 3060

gatgtggcct ttgtgaaaca ccagactgtc ccacagaaca ctgggggaaa aaaccctgat 3120

ccatgggcta agaatctgaa tgaaaaagac tatgagttgc tgtgccttga tggtaccagg 3180

aaacctgtgg aggagtatgc gaactgccac ctggccagag ccccgaatca cgctgtggtc 3240

acacggaaag ataaggaagc ttgcgtccac aagatattac gtcaacagca gcacctattt 3300

ggaagcaacg taactgactg ctcgggcaac ttttgtttgt tccggtcgga aaccaaggac 3360

cttctgttca gagatgacac agtatgtttg gccaaacttc atgacagaaa cacatatgaa 3420

aaatacttag gagaagaata tgtcaaggct gttggtaacc tgagaaaatg ctccacctca 3480

tcactcctgg aagcctgcac tttccgtaga ccttaa 3516

<210> 19
<211> 3546
<212> DNA
<213> Artificial Sequence

<220>
<223> gene sequence encoding FVII-GS11-Tf protein

<400> 19
atggtctccc aggccctcag gctcctctgc cttctgcttg ggcttcaggg ctgcctggct 60

gcagtcttcg taacccagga ggaagcccac ggcgtcctgc accggcgccg gcgcgccaac 120

gcgttcctgg aggagctgcg gccgggctcc ctggagaggg agtgcaagga ggagcagtgc 180

tccttcgagg aggcccggga gatcttcaag gacgcggaga ggacgaagct gttctggatt 240
tcttacagtg atggggacca gtgtgcctca agtccatgcc agaatggggg ctcctgcaag 300
gaccagctcc agtcctatat ctgcttctgc ctccctgcct tcgagggccg gaactgtgag 360
acgcacaagg atgaccagct gatctgtgtg aacgagaacg gcggctgtga gcagtactgc 420
agtgaccaca cgggcaccaa gcgctcctgt cggtgccacg aggggtactc tctgctggca 480
gacggggtgt cctgcacacc cacagttgaa tatccatgtg gaaaaatacc tattctagaa 540
aaaagaaatg ccagcaaacc ccaaggccga attgtggggg gcaaggtgtg ccccaaaggg 600
gagtgtccat ggcaggtcct gttgttggtg aatggagctc agttgtgtgg ggggaccctg 660
atcaacacca tctgggtggt ctccgcggcc cactgtttcg acaaaatcaa gaactggagg 720
aacctgatcg cggtgctggg cgagcacgac ctcagcgagc acgacgggga tgagcagagc 780
cggcgggtgg cgcaggtcat catccccagc acgtacgtcc cgggcaccac caaccacgac 840
atcgcgctgc tccgcctgca ccagcccgtg gtcctcactg accatgtggt gcccctctgc 900
ctgcccgaac ggacgttctc tgagaggacg ctggccttcg tgcgcttctc attggtcagc 960
ggctggggcc agctgctgga ccgtggcgcc acggccctgg agctcatggt cctcaacgtg 1020
ccccggctga tgacccagga ctgcctgcag cagtcacgga aggtgggaga ctccccaaat 1080
atcacggagt acatgttctg tgccggctac tcggatggca gcaaggactc ctgcaagggg 1140
gacagtggag gcccacatgc cacccactac cggggcacgt ggtacctgac gggcatcgtc 1200
agctggggcc agggctgcgc aaccgtgggc cactttgggg tgtacaccag ggtctcccag 1260
tacatcgagt ggctgcaaaa gctcatgcgc tcagagccac gcccaggagt cctcctgcga 1320
gccccatttc ccaccggtgg aggcggttca ggcggaggtg gctctggcgg tggcggatct 1380
ggcggaggtg gctctggcgg tggcggatct ggcggaggtg gctctggcgg tggcggatct 1440
ggcggaggtg gctctggcgg tggcggatct ggcggaggtg gctctggcgg tggcggatcc 1500
accggtgtcc ctgataaaac tgtgagatgg tgtgcagtgt cggagcatga ggccactaag 1560
tgccagagtt tccgcgacca tatgaaaagc gtcattccat ccgatggtcc cagtgttgct 1620
tgtgtgaaga aagcctccta ccttgattgc atcagggcca ttgcggcaaa cgaagcggat 1680
gctgtgacac tggatgcagg tttggtgtat gatgcttacc tggctcccaa taacctgaag 1740
cctgtggtgg cagagttcta tgggtcaaaa gaggatccac agactttcta ttatgctgtt 1800
gctgtggtga agaaggatag tggcttccag atgaaccagc ttcgaggcaa gaagtcctgc 1860
cacacgggtc taggcaggtc cgctgggtgg aacatcccca taggcttact ttactgtgac 1920
ttacctgagc cacgtaaacc tcttgagaaa gcagtggcca atttcttctc gggcagctgt 1980
gccccttgtg cggatgggac ggacttcccc cagctgtgtc aactgtgtcc agggtgtggc 2040
tgctccaccc ttaaccaata cttcggctac tcaggagcct tcaagtgtct gaaggatggt 2100
gctggggatg tggcctttgt caagcactcg actatatttg agaacttggc aaacaaggct 2160
gacagggacc agtatgagct gctttgcctg gacaacaccc ggaagccggt agatgaatac 2220

aaggactgcc acttggccca ggtcccttct cataccgtcg tggcccgaag tatgggcggc 2280

aaggaggact tgatctggga gcttctcaac caggcccagg aacattttgg caaagacaaa 2340

tcaaaagaat tccaactatt cagctctcct catgggaagg acctgctgtt taaggactct 2400

gcccacgggt ttttaaaagt cccccccagg atggatgcca agatgtacct gggctatgag 2460

tatgtcactg ccatccggaa tctacgggaa ggcacatgcc cagaagcccc aacagatgaa 2520

tgcaagcctg tgaagtggtg tgcgctgagc caccacgaga ggctcaagtg tgatgagtgg 2580

agtgttaaca gtgtagggaa aatagagtgt gtatcagcag agaccaccga agactgcatc 2640

gccaagatca tgaatggaga agctgatgcc atgagcttgg atggagggtt tgtctacata 2700

gcgggcaagt gtggtctggt gcctgtcttg gcagaaaact acaataagag cgataattgt 2760

gaggatacac cagaggcagg gtattttgct gtagcagtgg tgaagaaatc agcttctgac 2820

ctcacctggg acaatctgaa aggcaagaag tcctgccata cggcagttgg cagaaccgct 2880

ggctggaaca tccccatggg cctgctctac aataagatca accactgcag atttgatgaa 2940

tttttcagtg aaggttgtgc ccctgggtct aagaaagact ccagtctctg taagctgtgt 3000

atgggctcag gcctaaacct gtgtgaaccc aacaacaaag agggatacta cggctacaca 3060

ggcgctttca ggtgtctggt tgagaaggga gatgtggcct ttgtgaaaca ccagactgtc 3120

ccacagaaca ctgggggaaa aaaccctgat ccatgggcta agaatctgaa tgaaaaagac 3180

tatgagttgc tgtgccttga tggtaccagg aaacctgtgg aggagtatgc gaactgccac 3240

ctggccagag ccccgaatca cgctgtggtc acacggaaag ataaggaagc ttgcgtccac 3300

aagatattac gtcaacagca gcacctattt ggaagcaacg taactgactg ctcgggcaac 3360

ttttgtttgt tccggtcgga aaccaaggac cttctgttca gagatgacac agtatgtttg 3420

gccaaacttc atgacagaaa cacatatgaa aaatacttag gagaagaata tgtcaaggct 3480

gttggtaacc tgagaaaatg ctccacctca tcactcctgg aagcctgcac tttccgtaga 3540

ccttaa 3546

<210> 20
<211> 3576
<212> DNA
<213> Artificial Sequence

<220>
<223> gene sequence encoding FVII-GS13-Tf protein

<400> 20
atggtctccc aggccctcag gctcctctgc cttctgcttg ggcttcaggg ctgcctggct 60

gcagtcttcg taacccagga ggaagcccac ggcgtcctgc accggcgccg gcgcgccaac 120

gcgttcctgg aggagctgcg gccgggctcc ctggagaggg agtgcaagga ggagcagtgc 180

tccttcgagg aggcccggga gatcttcaag gacgcggaga ggacgaagct gttctggatt 240

tcttacagtg atggggacca gtgtgcctca agtccatgcc agaatggggg ctcctgcaag 300

gaccagctcc agtcctatat ctgcttctgc ctccctgcct tcgagggccg gaactgtgag 360

acgcacaagg atgaccagct gatctgtgtg aacgagaacg gcggctgtga gcagtactgc 420
agtgaccaca cgggcaccaa gcgctcctgt cggtgccacg aggggtactc tctgctggca 480
gacggggtgt cctgcacacc cacagttgaa tatccatgtg gaaaaatacc tattctagaa 540
aaaagaaatg ccagcaaacc ccaaggccga attgtggggg gcaaggtgtg ccccaaaggg 600
gagtgtccat ggcaggtcct gttgttggtg aatggagctc agttgtgtgg ggggaccctg 660
atcaacacca tctgggtggt ctccgcggcc cactgtttcg acaaaatcaa gaactggagg 720
aacctgatcg cggtgctggg cgagcacgac ctcagcgagc acgacgggga tgagcagagc 780
cggcgggtgg cgcaggtcat catccccagc acgtacgtcc cgggcaccac caaccacgac 840
atcgcgctgc tccgcctgca ccagcccgtg gtcctcactg accatgtggt gcccctctgc 900
ctgcccgaac ggacgttctc tgagaggacg ctggccttcg tgcgcttctc attggtcagc 960
ggctggggcc agctgctgga ccgtggcgcc acggccctgg agctcatggt cctcaacgtg 1020
ccccggctga tgacccagga ctgcctgcag cagtcacgga aggtgggaga ctccccaaat 1080
atcacggagt acatgttctg tgccggctac tcggatggca gcaaggactc ctgcaagggg 1140
gacagtggag gcccacatgc cacccactac cggggcacgt ggtacctgac gggcatcgtc 1200
agctggggcc agggctgcgc aaccgtgggc cactttgggg tgtacaccag ggtctcccag 1260
tacatcgagt ggctgcaaaa gctcatgcgc tcagagccac gcccaggagt cctcctgcga 1320
gccccatttc ccaccggtgg aggcggttca ggcggaggtg gctctggcgg tggcggatct 1380
ggcggaggtg gctctggcgg tggcggatct ggcggaggtg gctctggcgg tggcggatct 1440
ggcggaggtg gctctggcgg tggcggatct ggcggaggtg gctctggcgg tggcggatct 1500
ggcggaggtg gctctggcgg tggcggatcc accggtgtcc ctgataaaac tgtgagatgg 1560
tgtgcagtgt cggagcatga ggccactaag tgccagagtt tccgcgacca tatgaaaagc 1620
gtcattccat ccgatggtcc cagtgttgct tgtgtgaaga aagcctccta ccttgattgc 1680
atcagggcca ttgcggcaaa cgaagcggat gctgtgacac tggatgcagg tttggtgtat 1740
gatgcttacc tggctcccaa taacctgaag cctgtggtgg cagagttcta tgggtcaaaa 1800
gaggatccac agactttcta ttatgctgtt gctgtggtga agaaggatag tggcttccag 1860
atgaaccagc ttcgaggcaa gaagtcctgc cacacgggtc taggcaggtc cgctgggtgg 1920
aacatcccca taggcttact ttactgtgac ttacctgagc cacgtaaacc tcttgagaaa 1980
gcagtggcca atttcttctc gggcagctgt gccccttgtg cggatgggac ggacttcccc 2040
cagctgtgtc aactgtgtcc agggtgtggc tgctccaccc ttaaccaata cttcggctac 2100
tcaggagcct tcaagtgtct gaaggatggt gctggggatg tggcctttgt caagcactcg 2160
actatatttg agaacttggc aaacaaggct gacagggacc agtatgagct gctttgcctg 2220
gacaacaccc ggaagccggt agatgaatac aaggactgcc acttggccca ggtcccttct 2280
cataccgtcg tggcccgaag tatgggcggc aaggaggact tgatctggga gcttctcaac 2340
caggcccagg aacattttgg caaagacaaa tcaaaagaat tccaactatt cagctctcct 2400

catgggaagg acctgctgtt taaggactct gcccacgggt ttttaaaagt cccccccagg 2460

atggatgcca agatgtacct gggctatgag tatgtcactg ccatccggaa tctacgggaa 2520

ggcacatgcc cagaagcccc aacagatgaa tgcaagcctg tgaagtggtg tgcgctgagc 2580

caccacgaga ggctcaagtg tgatgagtgg agtgttaaca gtgtagggaa aatagagtgt 2640

gtatcagcag agaccaccga agactgcatc gccaagatca tgaatggaga agctgatgcc 2700

atgagcttgg atggagggtt tgtctacata gcgggcaagt gtggtctggt gcctgtcttg 2760

gcagaaaact acaataagag cgataattgt gaggatacac cagaggcagg gtattttgct 2820

gtagcagtgg tgaagaaatc agcttctgac ctcacctggg acaatctgaa aggcaagaag 2880

tcctgccata cggcagttgg cagaaccgct ggctggaaca tccccatggg cctgctctac 2940

aataagatca accactgcag atttgatgaa tttttcagtg aaggttgtgc ccctgggtct 3000

aagaaagact ccagtctctg taagctgtgt atgggctcag gcctaaacct gtgtgaaccc 3060

aacaacaaag agggatacta cggctacaca ggcgctttca ggtgtctggt tgagaaggga 3120

gatgtggcct ttgtgaaaca ccagactgtc ccacagaaca ctgggggaaa aaaccctgat 3180

ccatgggcta agaatctgaa tgaaaaagac tatgagttgc tgtgccttga tggtaccagg 3240

aaacctgtgg aggagtatgc gaactgccac ctggccagag ccccgaatca cgctgtggtc 3300

acacggaaag ataaggaagc ttgcgtccac aagatattac gtcaacagca gcacctattt 3360

ggaagcaacg taactgactg ctcgggcaac ttttgtttgt tccggtcgga aaccaaggac 3420

cttctgttca gagatgacac agtatgtttg gccaaacttc atgacagaaa cacatatgaa 3480

aaatacttag gagaagaata tgtcaaggct gttggtaacc tgagaaaatg ctccacctca 3540

tcactcctgg aagcctgcac tttccgtaga ccttaa 3576

<210> 21
<211> 3606
<212> DNA
<213> Artificial Sequence

<220>
<223> gene sequence encoding FVII-GS15-Tf protein

<400> 21
atggtctccc aggccctcag gctcctctgc cttctgcttg ggcttcaggg ctgcctggct 60

gcagtcttcg taacccagga ggaagcccac ggcgtcctgc accggcgccg gcgcgccaac 120

gcgttcctgg aggagctgcg gccgggctcc ctggagaggg agtgcaagga ggagcagtgc 180

tccttcgagg aggcccggga gatcttcaag gacgcggaga ggacgaagct gttctggatt 240

tcttacagtg atggggacca gtgtgcctca agtccatgcc agaatggggg ctcctgcaag 300

gaccagctcc agtcctatat ctgcttctgc ctccctgcct tcgagggccg gaactgtgag 360

acgcacaagg atgaccagct gatctgtgtg aacgagaacg gcggctgtga gcagtactgc 420

agtgaccaca cgggcaccaa gcgctcctgt cggtgccacg aggggtactc tctgctggca 480

gacggggtgt cctgcacacc cacagttgaa tatccatgtg gaaaaatacc tattctagaa 540
aaaagaaatg ccagcaaacc ccaaggccga attgtggggg gcaaggtgtg ccccaaaggg 600
gagtgtccat ggcaggtcct gttgttggtg aatggagctc agttgtgtgg ggggaccctg 660
atcaacacca tctgggtggt ctccgcggcc cactgtttcg acaaaatcaa gaactggagg 720
aacctgatcg cggtgctggg cgagcacgac ctcagcgagc acgacgggga tgagcagagc 780
cggcgggtgg cgcaggtcat catccccagc acgtacgtcc cgggcaccac caaccacgac 840
atcgcgctgc tccgcctgca ccagcccgtg gtcctcactg accatgtggt gcccctctgc 900
ctgcccgaac ggacgttctc tgagaggacg ctggccttcg tgcgcttctc attggtcagc 960
ggctggggcc agctgctgga ccgtggcgcc acggccctgg agctcatggt cctcaacgtg 1020
ccccggctga tgacccagga ctgcctgcag cagtcacgga aggtgggaga ctccccaaat 1080
atcacggagt acatgttctg tgccggctac tcggatggca gcaaggactc ctgcaagggg 1140
gacagtggag gcccacatgc cacccactac cggggcacgt ggtacctgac gggcatcgtc 1200
agctggggcc agggctgcgc aaccgtgggc cactttgggg tgtacaccag ggtctcccag 1260
tacatcgagt ggctgcaaaa gctcatgcgc tcagagccac gcccaggagt cctcctgcga 1320
gccccatttc ccaccggtgg aggcggttca ggcggaggtg gctctggcgg tggcggatct 1380
ggcggaggtg gctctggcgg tggcggatct ggcggaggtg gctctggcgg tggcggatct 1440
ggcggaggtg gctctggcgg tggcggatct ggcggaggtg gctctggcgg tggcggatct 1500
ggcggaggtg gctctggcgg tggcggatct ggcggaggtg gctctggcgg tggcggatcc 1560
accggtgtcc ctgataaaac tgtgagatgg tgtgcagtgt cggagcatga ggccactaag 1620
tgccagagtt tccgcgacca tatgaaaagc gtcattccat ccgatggtcc cagtgttgct 1680
tgtgtgaaga aagcctccta ccttgattgc atcagggcca ttgcggcaaa cgaagcggat 1740
gctgtgacac tggatgcagg tttggtgtat gatgcttacc tggctcccaa taacctgaag 1800
cctgtggtgg cagagttcta tgggtcaaaa gaggatccac agactttcta ttatgctgtt 1860
gctgtggtga agaaggatag tggcttccag atgaaccagc ttcgaggcaa gaagtcctgc 1920
cacacgggtc taggcaggtc cgctgggtgg aacatcccca taggcttact ttactgtgac 1980
ttacctgagc cacgtaaacc tcttgagaaa gcagtggcca atttcttctc gggcagctgt 2040
gccccttgtg cggatgggac ggacttcccc cagctgtgtc aactgtgtcc agggtgtggc 2100
tgctccaccc ttaaccaata cttcggctac tcaggagcct tcaagtgtct gaaggatggt 2160
gctggggatg tggcctttgt caagcactcg actatatttg agaacttggc aaacaaggct 2220
gacagggacc agtatgagct gctttgcctg gacaacaccc ggaagccggt agatgaatac 2280
aaggactgcc acttggccca ggtcccttct cataccgtcg tggcccgaag tatgggcggc 2340
aaggaggact tgatctggga gcttctcaac caggcccagg aacattttgg caaagacaaa 2400
tcaaaagaat tccaactatt cagctctcct catgggaagg acctgctgtt taaggactct 2460
gcccacgggt ttttaaaagt cccccccagg atggatgcca agatgtacct gggctatgag 2520

tatgtcactg ccatccggaa tctacgggaa ggcacatgcc cagaagcccc aacagatgaa 2580

tgcaagcctg tgaagtggtg tgcgctgagc caccacgaga ggctcaagtg tgatgagtgg 2640

agtgttaaca gtgtagggaa aatagagtgt gtatcagcag agaccaccga agactgcatc 2700

gccaagatca tgaatggaga agctgatgcc atgagcttgg atggagggtt tgtctacata 2760

gcgggcaagt gtggtctggt gcctgtcttg gcagaaaact acaataagag cgataattgt 2820

gaggatacac cagaggcagg gtattttgct gtagcagtgg tgaagaaatc agcttctgac 2880

ctcacctggg acaatctgaa aggcaagaag tcctgccata cggcagttgg cagaaccgct 2940

ggctggaaca tccccatggg cctgctctac aataagatca accactgcag atttgatgaa 3000

tttttcagtg aaggttgtgc ccctgggtct aagaaagact ccagtctctg taagctgtgt 3060

atgggctcag gcctaaacct gtgtgaaccc aacaacaaag agggatacta cggctacaca 3120

ggcgctttca ggtgtctggt tgagaaggga gatgtggcct ttgtgaaaca ccagactgtc 3180

ccacagaaca ctgggggaaa aaaccctgat ccatgggcta agaatctgaa tgaaaaagac 3240

tatgagttgc tgtgccttga tggtaccagg aaacctgtgg aggagtatgc gaactgccac 3300

ctggccagag ccccgaatca cgctgtggtc acacggaaag ataaggaagc ttgcgtccac 3360

aagatattac gtcaacagca gcacctattt ggaagcaacg taactgactg ctcgggcaac 3420

ttttgtttgt tccggtcgga aaccaaggac cttctgttca gagatgacac agtatgtttg 3480

gccaaacttc atgacagaaa cacatatgaa aaatacttag gagaagaata tgtcaaggct 3540

gttggtaacc tgagaaaatg ctccacctca tcactcctgg aagcctgcac tttccgtaga 3600

ccttaa 3606

<210> 22
<211> 3450
<212> DNA
<213> Artificial Sequence

<220>
<223> gene sequence encoding FVII-Helix-Tf protein

<400> 22
atggtctccc aggccctcag gctcctctgc cttctgcttg ggcttcaggg ctgcctggct 60

gcagtcttcg taacccagga ggaagcccac ggcgtcctgc accggcgccg gcgcgccaac 120

gcgttcctgg aggagctgcg gccgggctcc ctggagaggg agtgcaagga ggagcagtgc 180

tccttcgagg aggcccggga gatcttcaag gacgcggaga ggacgaagct gttctggatt 240

tcttacagtg atggggacca gtgtgcctca agtccatgcc agaatggggg ctcctgcaag 300

gaccagctcc agtcctatat ctgcttctgc ctccctgcct tcgagggccg gaactgtgag 360

acgcacaagg atgaccagct gatctgtgtg aacgagaacg gcggctgtga gcagtactgc 420

agtgaccaca cgggcaccaa gcgctcctgt cggtgccacg aggggtactc tctgctggca 480

gacggggtgt cctgcacacc cacagttgaa tatccatgtg gaaaaatacc tattctagaa 540

aaaagaaatg ccagcaaacc ccaaggccga attgtggggg gcaaggtgtg ccccaaaggg 600

gagtgtccat ggcaggtcct gttgttggtg aatggagctc agttgtgtgg ggggaccctg 660
atcaacacca tctgggtggt ctccgcggcc cactgtttcg acaaaatcaa gaactggagg 720
aacctgatcg cggtgctggg cgagcacgac ctcagcgagc acgacgggga tgagcagagc 780
cggcgggtgg cgcaggtcat catccccagc acgtacgtcc cgggcaccac caaccacgac 840
atcgcgctgc tccgcctgca ccagcccgtg gtcctcactg accatgtggt gcccctctgc 900
ctgcccgaac ggacgttctc tgagaggacg ctggccttcg tgcgcttctc attggtcagc 960
ggctggggcc agctgctgga ccgtggcgcc acggccctgg agctcatggt cctcaacgtg 1020
ccccggctga tgacccagga ctgcctgcag cagtcacgga aggtgggaga ctccccaaat 1080
atcacggagt acatgttctg tgccggctac tcggatggca gcaaggactc ctgcaagggg 1140
gacagtggag gcccacatgc cacccactac cggggcacgt ggtacctgac gggcatcgtc 1200
agctggggcc agggctgcgc aaccgtgggc cactttgggg tgtacaccag ggtctcccag 1260
tacatcgagt ggctgcaaaa gctcatgcgc tcagagccac gcccaggagt cctcctgcga 1320
gccccatttc ccaccggtgc tgaagctgca gccaaagaag ctgcagccaa agaggccgca 1380
gctaaggaag ccgcagcaaa agctaccggt gtccctgata aaactgtgag atggtgtgca 1440
gtgtcggagc atgaggccac taagtgccag agtttccgcg accatatgaa aagcgtcatt 1500
ccatccgatg gtcccagtgt tgcttgtgtg aagaaagcct cctaccttga ttgcatcagg 1560
gccattgcgg caaacgaagc ggatgctgtg acactggatg caggtttggt gtatgatgct 1620
tacctggctc ccaataacct gaagcctgtg gtggcagagt tctatgggtc aaaagaggat 1680
ccacagactt tctattatgc tgttgctgtg gtgaagaagg atagtggctt ccagatgaac 1740
cagcttcgag gcaagaagtc ctgccacacg ggtctaggca ggtccgctgg gtggaacatc 1800
cccataggct tactttactg tgacttacct gagccacgta aacctcttga gaaagcagtg 1860
gccaatttct tctcgggcag ctgtgcccct tgtgcggatg ggacggactt cccccagctg 1920
tgtcaactgt gtccagggtg tggctgctcc acccttaacc aatacttcgg ctactcagga 1980
gccttcaagt gtctgaagga tggtgctggg gatgtggcct ttgtcaagca ctcgactata 2040
tttgagaact tggcaaacaa ggctgacagg gaccagtatg agctgctttg cctggacaac 2100
acccggaagc cggtagatga atacaaggac tgccacttgg cccaggtccc ttctcatacc 2160
gtcgtggccc gaagtatggg cggcaaggag gacttgatct gggagcttct caaccaggcc 2220
caggaacatt ttggcaaaga caaatcaaaa gaattccaac tattcagctc tcctcatggg 2280
aaggacctgc tgtttaagga ctctgcccac gggtttttaa aagtcccccc caggatggat 2340
gccaagatgt acctgggcta tgagtatgtc actgccatcc ggaatctacg ggaaggcaca 2400
tgcccagaag ccccaacaga tgaatgcaag cctgtgaagt ggtgtgcgct gagccaccac 2460
gagaggctca agtgtgatga gtggagtgtt aacagtgtag ggaaaataga gtgtgtatca 2520
gcagagacca ccgaagactg catcgccaag atcatgaatg gagaagctga tgccatgagc 2580
ttggatggag ggtttgtcta catagcgggc aagtgtggtc tggtgcctgt cttggcagaa 2640

aactacaata agagcgataa ttgtgaggat acaccagagg cagggtattt tgctgtagca 2700

gtggtgaaga aatcagcttc tgacctcacc tgggacaatc tgaaaggcaa gaagtcctgc 2760

catacggcag ttggcagaac cgctggctgg aacatcccca tgggcctgct ctacaataag 2820

atcaaccact gcagatttga tgaatttttc agtgaaggtt gtgcccctgg gtctaagaaa 2880

gactccagtc tctgtaagct gtgtatgggc tcaggcctaa acctgtgtga acccaacaac 2940

aaagagggat actacggcta cacaggcgct ttcaggtgtc tggttgagaa gggagatgtg 3000

gcctttgtga aacaccagac tgtcccacag aacactgggg gaaaaaaccc tgatccatgg 3060

gctaagaatc tgaatgaaaa agactatgag ttgctgtgcc ttgatggtac caggaaacct 3120

gtggaggagt atgcgaactg ccacctggcc agagccccga atcacgctgt ggtcacacgg 3180

aaagataagg aagcttgcgt ccacaagata ttacgtcaac agcagcacct atttggaagc 3240

aacgtaactg actgctcggg caacttttgt ttgttccggt cggaaaccaa ggaccttctg 3300

ttcagagatg acacagtatg tttggccaaa cttcatgaca gaaacacata tgaaaaatac 3360

ttaggagaag aatatgtcaa ggctgttggt aacctgagaa aatgctccac ctcatcactc 3420

ctggaagcct gcactttccg tagaccttaa 3450

<210> 23
<211> 3426
<212> DNA
<213> Artificial Sequence

<220>
<223> gene sequence encoding FVII-GS1-T-Tf protein

<400> 23
atggtctccc aggccctcag gctcctctgc cttctgcttg ggcttcaggg ctgcctggct 60

gcagtcttcg taacccagga ggaagcccac ggcgtcctgc accggcgccg gcgcgccaac 120

gcgttcctgg aggagctgcg gccgggctcc ctggagaggg agtgcaagga ggagcagtgc 180

tccttcgagg aggcccggga gatcttcaag gacgcggaga ggacgaagct gttctggatt 240

tcttacagtg atggggacca gtgtgcctca agtccatgcc agaatggggg ctcctgcaag 300

gaccagctcc agtcctatat ctgcttctgc ctccctgcct tcgagggccg gaactgtgag 360

acgcacaagg atgaccagct gatctgtgtg aacgagaacg gcggctgtga gcagtactgc 420

agtgaccaca cgggcaccaa gcgctcctgt cggtgccacg aggggtactc tctgctggca 480

gacggggtgt cctgcacacc cacagttgaa tatccatgtg gaaaaatacc tattctagaa 540

aaaagaaatg ccagcaaacc ccaaggccga attgtggggg gcaaggtgtg ccccaaaggg 600

gagtgtccat ggcaggtcct gttgttggtg aatggagctc agttgtgtgg ggggaccctg 660

atcaacacca tctgggtggt ctccgcggcc cactgtttcg acaaaatcaa gaactggagg 720

aacctgatcg cggtgctggg cgagcacgac ctcagcgagc acgacgggga tgagcagagc 780

cggcgggtgg cgcaggtcat catccccagc acgtacgtcc cgggcaccac caaccacgac 840

atcgcgctgc tccgcctgca ccagcccgtg gtcctcactg accatgtggt gcccctctgc 900
ctgcccgaac ggacgttctc tgagaggacg ctggccttcg tgcgcttctc attggtcagc 960
ggctggggcc agctgctgga ccgtggcgcc acggccctgg agctcatggt cctcaacgtg 1020
ccccggctga tgacccagga ctgcctgcag cagtcacgga aggtgggaga ctccccaaat 1080
atcacggagt acatgttctg tgccggctac tcggatggca gcaaggactc ctgcaagggg 1140
gacagtggag gcccacatgc cacccactac cggggcacgt ggtacctgac gggcatcgtc 1200
agctggggcc agggctgcgc aaccgtgggc cactttgggg tgtacaccag ggtctcccag 1260
tacatcgagt ggctgcaaaa gctcatgcgc tcagagccac gcccaggagt cctcctgcga 1320
gccccatttc ccaccggtgg aggcggatcc ctggtgccgc gcggcagcgg aggcggttca 1380
accggtgtcc ctgataaaac tgtgagatgg tgtgcagtgt cggagcatga ggccactaag 1440
tgccagagtt tccgcgacca tatgaaaagc gtcattccat ccgatggtcc cagtgttgct 1500
tgtgtgaaga aagcctccta ccttgattgc atcagggcca ttgcggcaaa cgaagcggat 1560
gctgtgacac tggatgcagg tttggtgtat gatgcttacc tggctcccaa taacctgaag 1620
cctgtggtgg cagagttcta tgggtcaaaa gaggatccac agactttcta ttatgctgtt 1680
gctgtggtga agaaggatag tggcttccag atgaaccagc ttcgaggcaa gaagtcctgc 1740
cacacgggtc taggcaggtc cgctgggtgg aacatcccca taggcttact ttactgtgac 1800
ttacctgagc cacgtaaacc tcttgagaaa gcagtggcca atttcttctc gggcagctgt 1860
gccccttgtg cggatgggac ggacttcccc cagctgtgtc aactgtgtcc agggtgtggc 1920
tgctccaccc ttaaccaata cttcggctac tcaggagcct tcaagtgtct gaaggatggt 1980
gctggggatg tggcctttgt caagcactcg actatatttg agaacttggc aaacaaggct 2040
gacagggacc agtatgagct gctttgcctg gacaacaccc ggaagccggt agatgaatac 2100
aaggactgcc acttggccca ggtcccttct cataccgtcg tggcccgaag tatgggcggc 2160
aaggaggact tgatctggga gcttctcaac caggcccagg aacattttgg caaagacaaa 2220
tcaaaagaat tccaactatt cagctctcct catgggaagg acctgctgtt taaggactct 2280
gcccacgggt ttttaaaagt cccccccagg atggatgcca agatgtacct gggctatgag 2340
tatgtcactg ccatccggaa tctacgggaa ggcacatgcc cagaagcccc aacagatgaa 2400
tgcaagcctg tgaagtggtg tgcgctgagc caccacgaga ggctcaagtg tgatgagtgg 2460
agtgttaaca gtgtagggaa aatagagtgt gtatcagcag agaccaccga agactgcatc 2520
gccaagatca tgaatggaga agctgatgcc atgagcttgg atggagggtt tgtctacata 2580
gcgggcaagt gtggtctggt gcctgtcttg gcagaaaact acaataagag cgataattgt 2640
gaggatacac cagaggcagg gtattttgct gtagcagtgg tgaagaaatc agcttctgac 2700
ctcacctggg acaatctgaa aggcaagaag tcctgccata cggcagttgg cagaaccgct 2760
ggctggaaca tccccatggg cctgctctac aataagatca accactgcag atttgatgaa 2820
tttttcagtg aaggttgtgc ccctgggtct aagaaagact ccagtctctg taagctgtgt 2880

atgggctcag gcctaaacct gtgtgaaccc aacaacaaag agggatacta cggctacaca 2940

ggcgctttca ggtgtctggt tgagaaggga gatgtggcct ttgtgaaaca ccagactgtc 3000

ccacagaaca ctgggggaaa aaaccctgat ccatgggcta agaatctgaa tgaaaaagac 3060

tatgagttgc tgtgccttga tggtaccagg aaacctgtgg aggagtatgc gaactgccac 3120

ctggccagag ccccgaatca cgctgtggtc acacggaaag ataaggaagc ttgcgtccac 3180

aagatattac gtcaacagca gcacctattt ggaagcaacg taactgactg ctcgggcaac 3240

ttttgtttgt tccggtcgga aaccaaggac cttctgttca gagatgacac agtatgtttg 3300

gccaaacttc atgacagaaa cacatatgaa aaatacttag gagaagaata tgtcaaggct 3360

gttggtaacc tgagaaaatg ctccacctca tcactcctgg aagcctgcac tttccgtaga 3420

ccttaa 3426

<210> 24
<211> 3417
<212> DNA
<213> Artificial Sequence

<220>
<223> gene sequence encoding FVII-GS1-T-Tf(M3) protein

<400> 24
atggtctccc aggccctcag gctcctctgc cttctgcttg ggcttcaggg ctgcctggct 60

gcagtcttcg taacccagga ggaagcccac ggcgtcctgc accggcgccg gcgcgccaac 120

gcgttcctgg aggagctgcg gccgggctcc ctggagaggg agtgcaagga ggagcagtgc 180

tccttcgagg aggcccggga gatcttcaag gacgcggaga ggacgaagct gttctggatt 240

tcttacagtg atggggacca gtgtgcctca agtccatgcc agaatggggg ctcctgcaag 300

gaccagctcc agtcctatat ctgcttctgc ctccctgcct tcgagggccg gaactgtgag 360

acgcacaagg atgaccagct gatctgtgtg aacgagaacg gcggctgtga gcagtactgc 420

agtgaccaca cgggcaccaa gcgctcctgt cggtgccacg aggggtactc tctgctggca 480

gacggggtgt cctgcacacc cacagttgaa tatccatgtg gaaaaatacc tattctagaa 540

aaaagaaatg ccagcaaacc ccaaggccga attgtggggg gcaaggtgtg ccccaaaggg 600

gagtgtccat ggcaggtcct gttgttggtg aatggagctc agttgtgtgg ggggaccctg 660

atcaacacca tctgggtggt ctccgcggcc cactgtttcg acaaaatcaa gaactggagg 720

aacctgatcg cggtgctggg cgagcacgac ctcagcgagc acgacgggga tgagcagagc 780

cggcgggtgg cgcaggtcat catccccagc acgtacgtcc cgggcaccac caaccacgac 840

atcgcgctgc tccgcctgca ccagcccgtg gtcctcactg accatgtggt gcccctctgc 900

ctgcccgaac ggacgttctc tgagaggacg ctggccttcg tgcgcttctc attggtcagc 960

ggctggggcc agctgctgga ccgtggcgcc acggccctgg agctcatggt cctcaacgtg 1020

ccccggctga tgacccagga ctgcctgcag cagtcacgga aggtgggaga ctccccaaat 1080

atcacggagt acatgttctg tgccggctac tcggatggca gcaaggactc ctgcaagggg 1140

gacagtggag gcccacatgc cacccactac cggggcacgt ggtacctgac gggcatcgtc 1200
agctggggcc agggctgcgc aaccgtgggc cactttgggg tgtacaccag ggtctcccag 1260
tacatcgagt ggctgcaaaa gctcatgcgc tcagagccac gcccaggagt cctcctgcga 1320
gccccatttc ccggtggagg cggatccctg gtgccgcgcg gcagcggagg cggttcagtc 1380
cctgataaaa ctgtgagatg gtgtgcagtg tcggagcatg aggccactaa gtgccagagt 1440
ttccgcgacc atatgaaaag cgtcattcca tccgatggtc ccagtgttgc ttgtgtgaag 1500
aaagcctcct accttgattg catcagggcc attgcggcaa acgaagcgga tgctgtgaca 1560
ctggatgcag gtttggtgta tgatgcttac ctggctccca ataacctgaa gcctgtggtg 1620
gcagagttct atgggtcaaa agaggatcca cagactttct attatgctgt tgctgtggtg 1680
aagaaggata gtggcttcca gatgaaccag cttcgaggca agaagtcctg ccacacgggt 1740
ctaggcaggt ccgctgggtg gaacatcccc ataggcttac tttactgtga cttacctgag 1800
ccacgtaaac ctcttgagaa agcagtggcc aatttcttct cgggcagctg tgccccttgt 1860
gcggatggga cggacttccc ccagctgtgt caactgtgtc cagggtgtgg ctgctccacc 1920
cttaaccaat acttcggcta ctcaggagcc ttcaagtgtc tgaaggatgg tgctggggat 1980
gtggcctttg tcaagcactc gactatattt gagaacttgg caaacaaggc tgacagggac 2040
cagtatgagc tgctttgcct ggacaacacc cggaagccgg tagatgaata caaggactgc 2100
cacttggccc aggtcccttc tcataccgtc gtggcccgaa gtatgggcgg caaggaggac 2160
ttgatctggg agcttctcaa ccaggcccag gaacattttg gcaaagacaa atcaaaagaa 2220
ttccaactat tcagctctcc tcatgggaag gacctgctgt ttaaggactc tgcccacggg 2280
tttttaaaag tcccccccag gatggatgcc aagatgtacc tgggctatga gtatgtcact 2340
gccatccgga atctacggga aggcacatgc ccagaagccc caacagatga atgcaagcct 2400
gtgaagtggt gtgcgctgag ccaccacgag aggctcaagt gtgatgagtg gagtgttaac 2460
agtgtaggga aaatagagtg tgtatcagca gagaccaccg aagactgcat cgccaagatc 2520
atgaatggag aagctgatgc catgagcttg gatggagggt ttgtctacat agcgggcaag 2580
tgtggtctgg tgcctgtctt ggcagaaaac tacaataaga gcgataattg tgaggataca 2640
ccagaggcag ggtattttgc tgtagcagtg gtgaagaaat cagcttctga cctcacctgg 2700
gacaatctga aaggcaagaa gtcctgccat acggcagttg gcagaaccgc tggctggaac 2760
atccccatgg gcctgctcta caataagatc aaccactgca gatttgatga atttttcagt 2820
gaaggttgtg cccctgggtc taagaaagac tccagtctct gtaagctgtg tatgggctca 2880
ggcctaaacc tgtgtgaacc caacaacaaa gagggatact acggctacac aggcgctttc 2940
aggtgtctgg ttgagaaggg agatgtggcc tttgtgaaac accagactgt cccacagaac 3000
actgggggaa aaaaccctga tccatgggct aagaatctga atgaaaaaga ctatgagttg 3060
ctgtgccttg atggtaccag gaaacctgtg gaggagtatg cgaactgcca cctggccaga 3120
gccccgaatc acgctgtggt cacacggaaa gataaggaag cttgcgtcca caagatatta 3180

cgtcaacagc agcacctatt tggaagcaac gtaactgact gctcgggcaa cttttgtttg 3240

ttccggtcgg aaaccaagga ccttctgttc agagatgaca cagtatgttt ggccaaactt 3300

catgacagaa acacatatga aaaatactta ggagaagaat atgtcaaggc tgttggtaac 3360

ctgagaaaat gctccacctc atcactcctg gaagcctgca ctttccgtag accttaa 3417

<210> 25
<211> 25
<212> DNA
<213> Artificial Sequence

<220>
<223> FVII-F primer

<400> 25
aggggcagca ctgcagagat ttcat 25

<210> 26
<211> 25
<212> DNA
<213> Artificial Sequence

<220>
<223> FVII-R primer

<400> 26
tatgggattt ggtgccagga cagtt 25

<210> 27
<211> 31
<212> DNA
<213> Artificial Sequence

<220>
<223> FVII-S1 primer

<400> 27
aattgctagc atggtctccc aggccctcag g 31

<210> 28
<211> 31
<212> DNA
<213> Artificial Sequence

<220>
<223> FVII-AS1 primer

<400> 28
aattaccggt gggaaatggg gctcgcagga g 31

<210> 29
<211> 33
<212> DNA
<213> Artificial Sequence

<220>

<223>    Tf-S1 primer

<400>    29
atataccggt gtccctgata aaactgtgag atg                                    33

<210>    30
<211>    34
<212>    DNA
<213>    Artificial Sequence

<220>
<223>    Tf-AS1 primer

<400>    30
aattctcgag ttaaggtcta cggaaagtgc aggc                                   34

<210>    31
<211>    36
<212>    DNA
<213>    Artificial Sequence

<220>
<223>    GS-FV-AS1 primer

<400>    31
ggatccgcct ccaccgggaa atggggctcg caggag                                 36

<210>    32
<211>    40
<212>    DNA
<213>    Artificial Sequence

<220>
<223>    GS-Tf-S1 primer

<400>    32
ggtggaggcg gatccgtccc tgataaaact gtgagatggt                             40

<210>    33
<211>    48
<212>    DNA
<213>    Artificial Sequence

<220>
<223>    GS3-S primer

<400>    33
ccggtggagg cggttcaggc ggaggtggct ctggcggtgg cggatcca                    48

<210>    34
<211>    48
<212>    DNA
<213>    Artificial Sequence

<220>
<223>    GS3-AS primer

<400>    34
acctccgcca agtccgcctc caccgagacc gccaccgcct aggtggcc                48

<210>    35
<211>    50
<212>    DNA
<213>    Artificial Sequence

<220>
<223>    GS-2 linker unit

<400>    35
tattagatct ggcggaggtg gctctggcgg tggcggatcc accggtatta              50

<210>    36
<211>    60
<212>    DNA
<213>    Artificial Sequence

<220>
<223>    dsGS1-T linker

<400>    36
aattaccggt ggaggcggat ccctggtgcc gcgcggcagc ggaggcggtt caaccggtat    60

                                                                     60

<210>    37
<211>    58
<212>    DNA
<213>    Artificial Sequence

<220>
<223>    Helix linker S primer

<400>    37
aattaccggt gctgaagctg cagccaaaga agctgcagcc aaagaggccg cagctaag      58

<210>    38
<211>    46
<212>    DNA
<213>    Artificial Sequence

<220>
<223>    Helix linker AS primer

<400>    38
ttataccggt agcttttgct gcggcttcct tagctgcggc ctcttt                  46

<210>    39
<211>    31
<212>    DNA
<213>    Artificial Sequence

<220>
<223>    Albumin-S primer

<400>    39

gtgggatccg atgcacacaa gagtgaggtt g                                    31

<210>   40
<211>   35
<212>   DNA
<213>   Artificial Sequence

<220>
<223>   Albumin-AS primer

<400>   40
cacggatccc tataagccta aggcagcttg acttg                                35

<210>   41
<211>   55
<212>   DNA
<213>   Artificial Sequence

<220>
<223>   oligonucleotide 1

<400>   41
gtgctcgagc gggggatctg gcgggtctgg aggctctgga gggtcgggag gctct          55

<210>   42
<211>   74
<212>   DNA
<213>   Artificial Sequence

<220>
<223>   oligonucleotide 2

<400>   42
caagggccct tatcaggatc ccgaccctcc agacccgcca gatcccccag agcctccaga     60

gcctcccgac cctc                                                       74

<210>   43
<211>   33
<212>   DNA
<213>   Artificial Sequence

<220>
<223>   mut FVII(XhoI)-S primer

<400>   43
gagccccatt tccctcgagc ccagcagccc tgg                                  33

<210>   44
<211>   33
<212>   DNA
<213>   Artificial Sequence

<220>
<223>   mut FVII(XhoI)-AS primer

<400>   44
ccagggctgc tgggctcgag ggaaatgggg ctc                                  33

<210>    45
<211>    31
<212>    PRT
<213>    Artificial Sequence

<220>
<223>    GS linker (EP1816201)

<400>    45
Ser Ser Gly Gly Ser Gly Gly Ser Gly Gly Ser Gly Gly Ser Gly Gly
1               5                   10                  15

Ser Gly Gly Ser Gly Gly Ser Gly Gly Ser Gly Gly Ser Gly Ser
            20                  25                  30

<210>    46
<211>    25
<212>    DNA
<213>    Artificial Sequence

<220>
<223>    Primer Nhe-Tf

<400>    46
aattgctagc atgaggctcg ccgtg                                          25

<210>    47
<211>    28
<212>    DNA
<213>    Artificial Sequence

<220>
<223>    Primer Tf-Age

<400>    47
aattaccggt aggtctacgg aaagtgca                                       28

<210>    48
<211>    25
<212>    DNA
<213>    Artificial Sequence

<220>
<223>    Primer Age-VII

<400>    48
aattaccggt gccaacgcgt tcctg                                          25

<210>    49
<211>    28
<212>    DNA
<213>    Artificial Sequence

<220>
<223>    Primer VII-Xho

<400>    49
aattctcgag ttagggaaat ggggctcg                                          28


<210>    50
<211>    32
<212>    DNA
<213>    Artificial Sequence

<220>
<223>    Primer TG del-S


<400>    50
cagcggaggc ggttcagtcc ctgataaaac tg                                     32


<210>    51
<211>    32
<212>    DNA
<213>    Artificial Sequence

<220>
<223>    Primer TG del-AS


<400>    51
cagttttatc agggactgaa ccgcctccgc tg                                     32


<210>    52
<211>    29
<212>    DNA
<213>    Artificial Sequence

<220>
<223>    Primer T del-S


<400>    52
cgagccccat ttcccggtgg aggcggatc                                         29


<210>    53
<211>    29
<212>    DNA
<213>    Artificial Sequence

<220>
<223>    Primer T del-AS


<400>    53
gatccgcctc caccgggaaa tggggctcg                                         29

## Description

### [Technical Field]

The present invention relates to a method of isolating and purifying a fusion protein containing factor VII (FVII), and more particularly, to a method of isolating and purifying a fusion protein including FVII and transferrin to a high degree of purity.

### [Background Art]

Blood coagulation factor VII (hereinafter referred to as 'factor VII' or 'FVII') is a plasma protein which is dependent on vitamin K, and it plays a key role in the initiation of blood coagulation. In blood and tissues, over 50 substances are involved in the blood coagulation. In this case, the substances taking part in the blood coagulation are referred to as procoagulants, and the substances preventing the blood coagulation are referred to as anticoagulants. Two conflicting groups of the substances in blood and tissues are always in equilibrium, but the anticoagulants are generally predominant to prevent blood from coagulating. However, when blood vessels are damaged, the procoagulants become predominant over the anticoagulants in this affected part. In this case, prothrombin, which is one of the plasma proteins in blood produced in the liver by the action of vitamin K, is converted into thrombin by the action of calcium ions or platelets. Then, the thrombin functions to convert fibrinogen to fibrin, which leads to coagulation of blood.

Depending on activation mechanisms, pathways for the blood coagulation are divided into an extrinsic pathway starting from the damage of blood vessel walls or their surrounding tissues and an intrinsic pathway starting from blood itself. Between these two pathways, the extrinsic pathway is initiated as tissue factors (TFs) present in cell membranes are exposed to circulating blood, when blood vessel walls are damaged, thereby to form a complex with FVII or active factor VII (FVIIa: present in blood at an amount corresponding to approximately 1% of the total mass of FVII proteins) present in blood. Since such a complex has catalytic activities, it acts on factor X (FX) present on the cell surface so that the FX is converted into activated factor X (FXa). Then, the FXa results in a blood coagulation cascade to convert factor IX (FIX) into active factor IX (FIXa).

FVII is a single-chain glycoprotein which consists of 406 amino acids, has a molecular weight of approximately 50 kDa, and is secreted by the liver into the blood stream as inert zymogen. FVII contains four domains: an amino-terminal-carboxyglutamate (Glu) domain, two epidermal growth factor (EGF)-like domains, and a serine protease domain (Hagen FS et al., Proc. Natl. Acad. Sci., USA, 83(8): 2412-2416, 1986). FVII is converted into its active form FVIIa by the proteolysis of a single peptide bond at Arg152-Ile153, leading to the formation of two polypeptide chains, an N-terminal light chain (24 kDa) and a C-terminal heavy chain (28 kDa), which are linked to each other by a disulfide bond. Factor VII is present in the plasma at a concentration of 500 ng/mL. 1% (i.e., 5 ng/mL) of the Factor VII is present as Factor VIIa.

FVII has the shortest plasma half-life among the protein in the serum, and thus it is a problem that the FVII should be repeatedly administered so as to effectively stop the bleeding. To solve the above problems, techniques capable of enhancing the half-life of FVII to improve the patients' convenience and to enhance the efficiency, for example, PEGylation, carboxyl terminal peptide (CTP), and albumin fusion techniques, have been studied so far.

Particularly, the present inventors have proposed a type of a fusion protein in which FVII is fused to transferrin as a way to enhance the *in vivo* half-life of FVII (see Korean Unexamined Patent Publication No. 10-2011-0133454). Since the FVII-transferrin fusion protein has a longer half-life *in vivo* than wild-type FVII and also retains biological activities of FVII, it may be able to replace the use of FVII for the treatment.

The fusion protein may be expressed in host cells (e.g., CHO cells, etc.) using a recombinant vector having a DNA sequence coding for the fusion protein inserted thereto. However, a method of isolating and purifying the expressed protein remains to be established. In the prior art, methods of isolating and purifying a blood coagulation factor itself using column chromatography were studied, but the purity of the isolated and purified protein was very low, which makes it difficult to apply them to the isolation and purification of the fusion protein.

### [Disclosure]

### [Technical Problem]

Therefore, the present invention is designed to solve the problems of the prior art, and therefore it is an object of the present invention to provide a method of isolating and purifying a fusion protein, which includes factor VII expressed by a genetic engineering methods, to a high degree of purity.

### [Technical Solution]

A method of isolating and purifying a fusion protein containing factor VII is provide, which comprises 1) isolating and purifying the fusion protein containing factor VII expressed in animal cells by affinity chromatography using a resin having a structure represented by Formula 1 as a stationary phase or by mixed-mode chromatography using a ceramic fluoroapatite gel (Ca₁₀(PO₄)₆F₂) as a stationary phase, and 2) further isolating and purifying the fusion protein by anion exchange chromatography using Q-sepharose FF gel as a stationary phase.

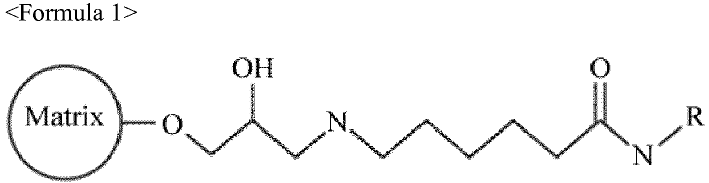

In Formula 1, the matrix is cross-linked agarose, and R is a factor VII binding protein serving as a ligand.

### [Advantageous Effects]

Because the present invention provides a method capable of isolating and purifying a recombinant fusion protein containing factor VII to a high degree of purity, the method according to one exemplary embodiment of the invention can be useful in manufacturing a pharmaceutical preparation including the factor VII that can be used in cases in which copious bleeding occurs such as surgery.

### [Description of Drawings]

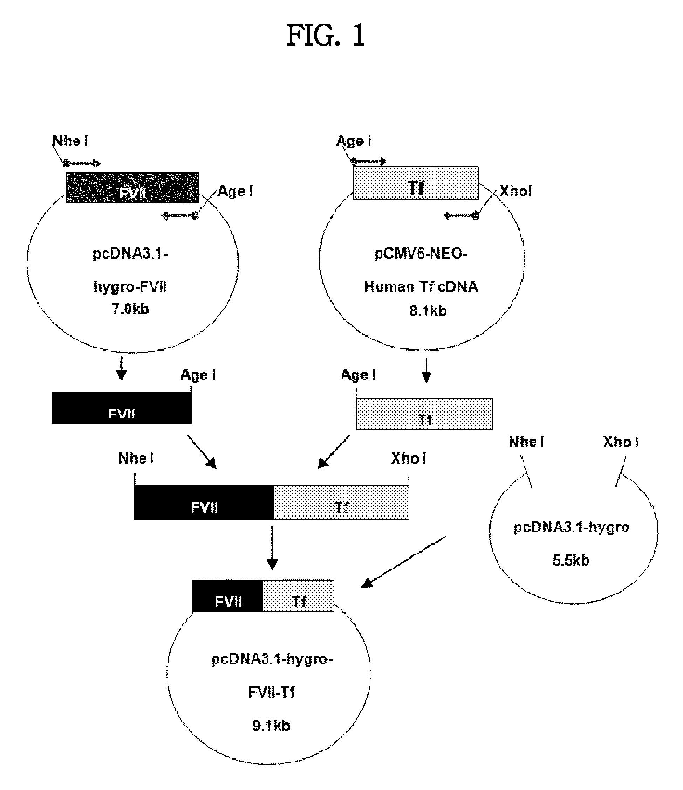

FIG. 1 is a schematic diagram showing a cloning procedure for constructing a FVII-Tf expression vector from a vector containing a cDNA coding for a FVII and a vector containing a cDNA coding for a transferrin (Tf).

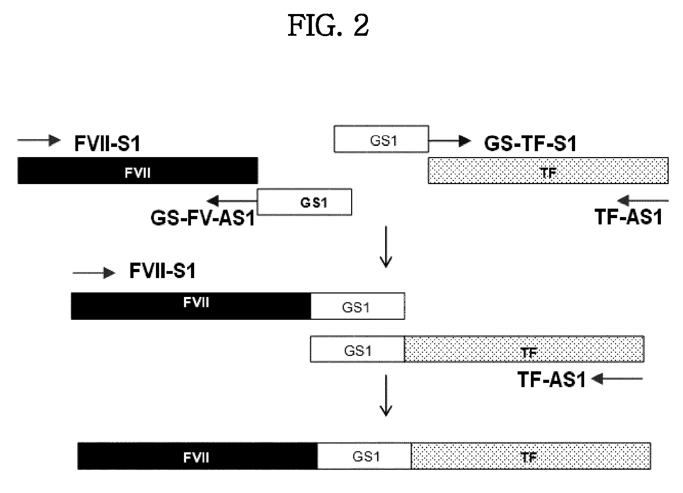

FIG. 2 is a schematic diagram showing a process of preparing a FVII-GS1 (linker)-Tf expression vector using an overlapping polymerase chain reaction (PCR).

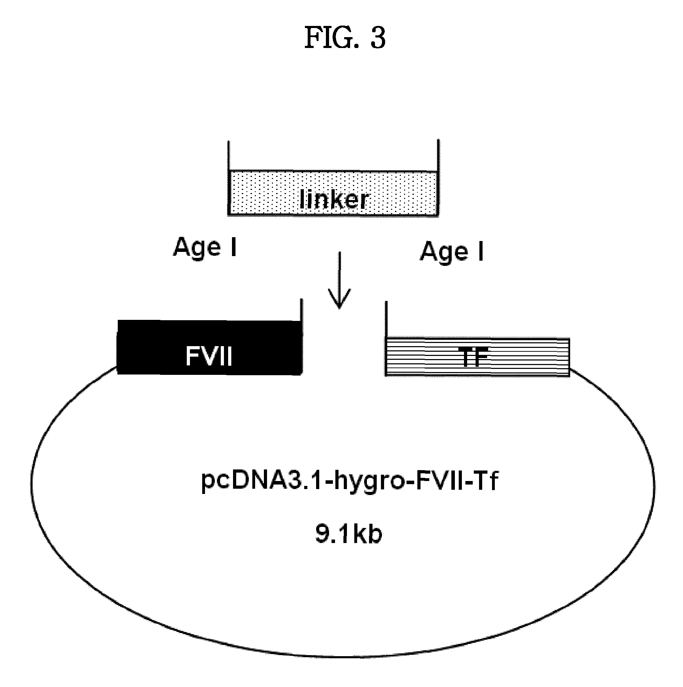

FIG. 3 is a schematic diagram showing a process of preparing a FVII-GS linker-Tf expression vector containing GS3, GS5, GS7, GS9, GS11, GS13, GS15, or GS-1-T as a linker.

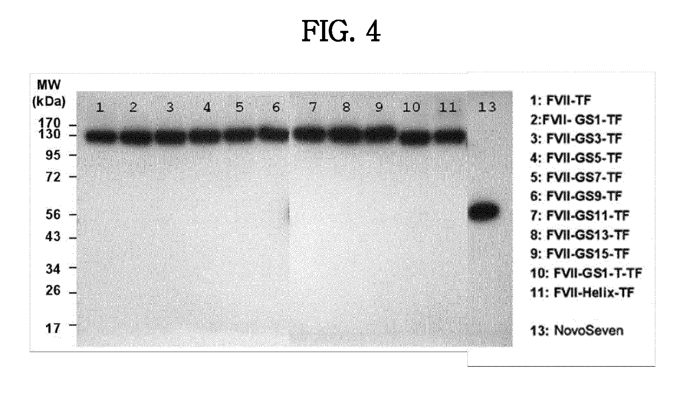

FIG. 4 shows the results obtained by confirming the presence of FVII-Tf, FVII-GS1-Tf, FVII-GS3-Tf, FVII-GS5-Tf, FVII-GS7-Tf, FVII-GS9-Tf, FVII-GS11-Tf, FVII-GS 13-Tf, FVII-GS 15-Tf, FVII-GS 1-T-Tf, FVII-Helix-Tf, which are the fusion proteins according to one exemplary embodiment of the present invention, and FVII (NovoSeven™) by Western blotting.

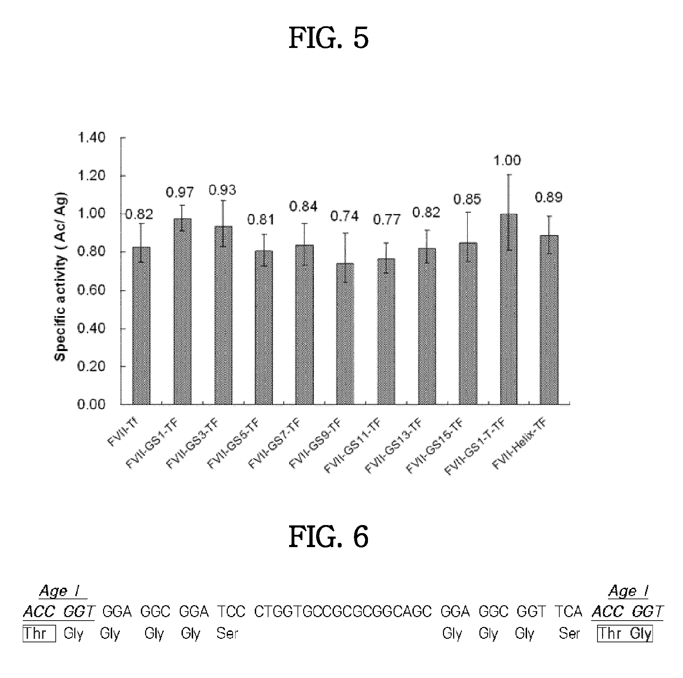

FIG. 5 is a graph showing the specific activities of FVII-Tf, FVII-GS1-Tf, FVII-GS3-Tf, FVII-GS5-Tf, FVII-GS7-Tf, FVII-GS9-Tf, FVII-GS11-Tf, FVII-GS13-Tf, FVII-GS 15-Tf, FVII-GS 1-T-Tf, and FVII-Helix-Tf, which are the fusion proteins according to one exemplary embodiment of the present invention.
FIG. 6 is a diagram showing a structure of the fusion protein, FVII-GS1-T-Tf, according to one exemplary embodiment of the present invention, which includes a linker and a restriction endonuclease recognition sequences flanking at both ends thereof.

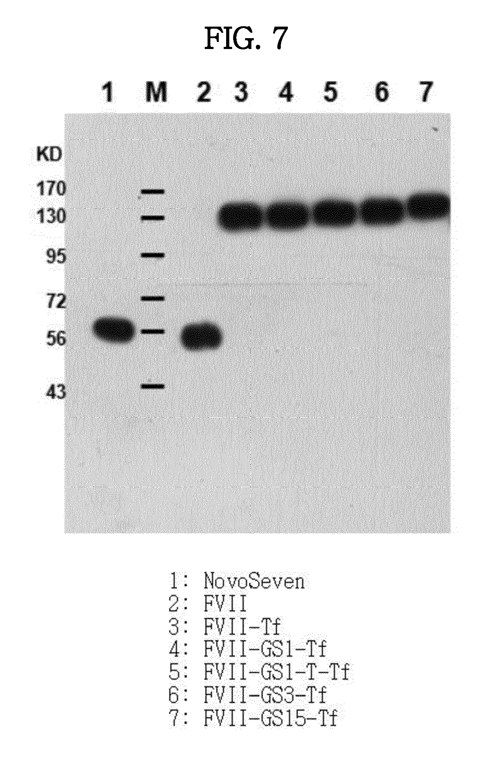

FIG. 7 is a diagram showing the Western blotting results of FVII-Tf, FVII-GS 1-Tf, FVII-GS 1-T-Tf, FVII-GS3-Tf, FVII-GS 15-Tf, which are the purified fusion proteins according to one exemplary embodiment of the present invention, NovoSeven^{®}, and FVII.

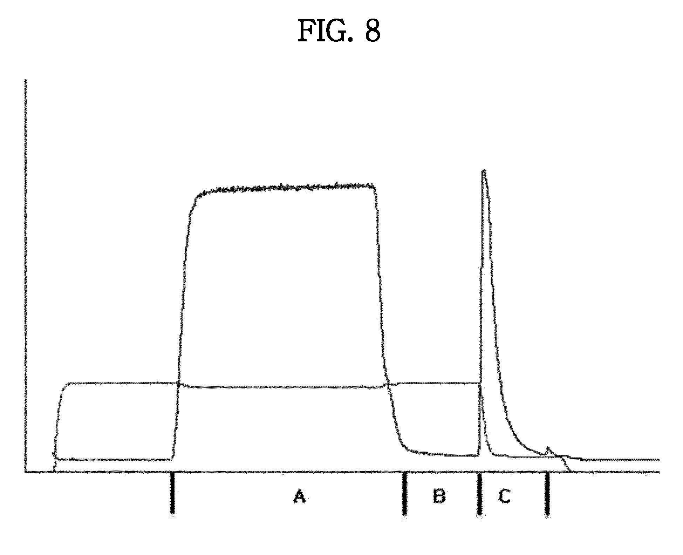

FIG. 8 is a chromatogram showing the light absorbance measured at a UV wavelength of 280 nm for the recombinant FVII fusion protein (FVII-GSI-T-Tf) according to one exemplary embodiment of the present invention after the recombinant FVII fusion protein (FVII-GS1-T-Tf) is isolated and purified under the elution conditions described in Example <9-2> by affinity chromatography using an XK16/20 VIISelect as a stationary phase.

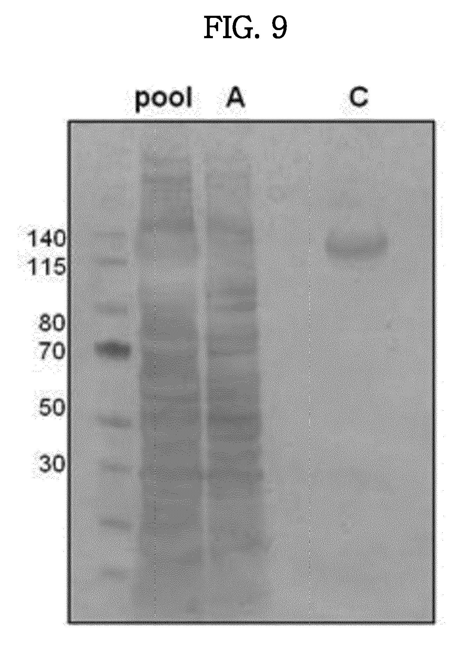

FIG. 9 shows the results analyzed for the recombinant FVII fusion protein (FVII-GS1-T-Tf) according to one exemplary embodiment of the present invention by SDS-PAGE after the recombinant FVII fusion protein (FVII-GS1-T-Tf) is isolated and purified under the elution conditions described in Example <9-2> by affinity chromatography using an XK16/20 VIISelect as a stationary phase.

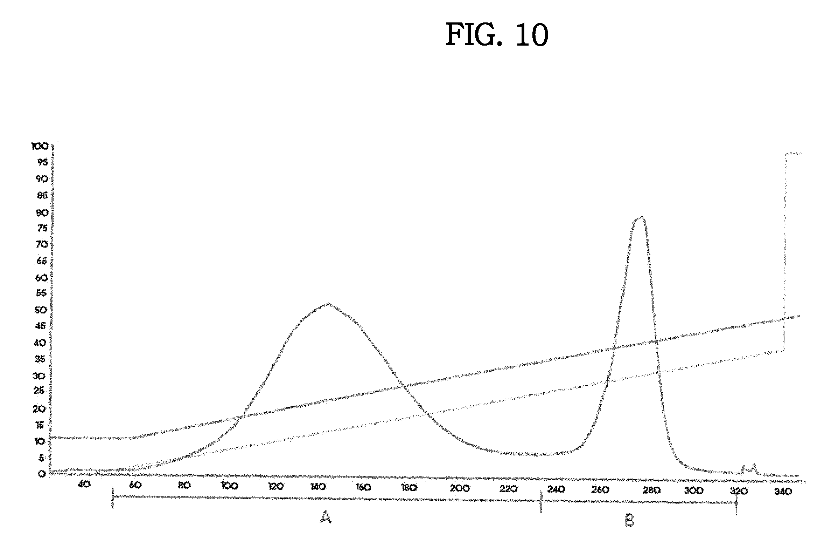

FIG. 10 is a chromatogram showing the light absorbance measured at a UV wavelength of 280 nm for the recombinant FVII fusion protein (FVII-GSI-T-Tf) according to one exemplary embodiment of the present invention after the recombinant FVII fusion protein (FVII-GS1-T-Tf) is isolated and purified under the elution conditions described in Example <9-2> by affinity chromatography using an XK16/20 VIISelect as a stationary phase and further isolated and purified by anion exchange chromatography using Q-sepharose FF as a stationary phase.

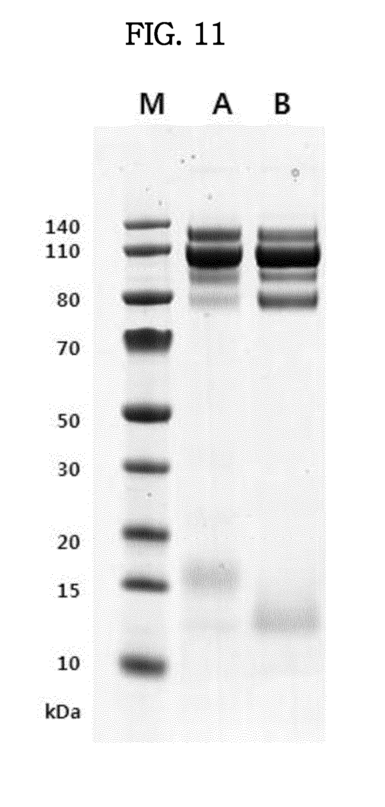

FIG. 11 shows the results analyzed for the recombinant FVII fusion protein (FVII-GS1-T-Tf) according to one exemplary embodiment of the present invention by SDS-PAGE after the recombinant FVII fusion protein (FVII-GS1-T-Tf) is isolated and purified under the elution conditions described in Example <9-2> by affinity chromatography using an XK16/20 VIISelect as a stationary phase and further isolated and purified by anion exchange chromatography using Q-sepharose FF as a stationary phase.

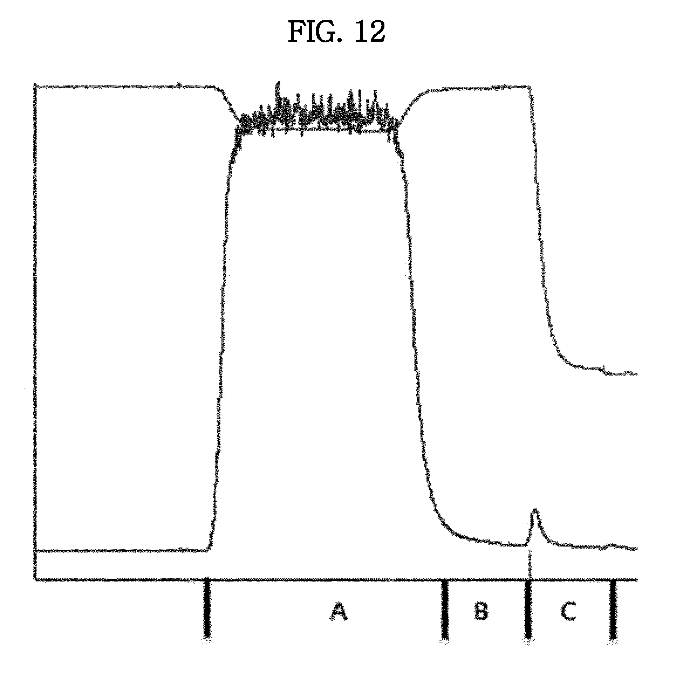

FIG. 12 is a chromatogram showing the light absorbance measured at a UV wavelength of 280 nm for the recombinant FVII fusion protein (FVII-GSI-T-Tf) according to one exemplary embodiment of the present invention after the recombinant FVII fusion protein (FVII-GS1-T-Tf) is isolated and purified under the elution conditions described in Comparative Example 1 by affinity chromatography using an XK16/20 VIISelect as a stationary phase.

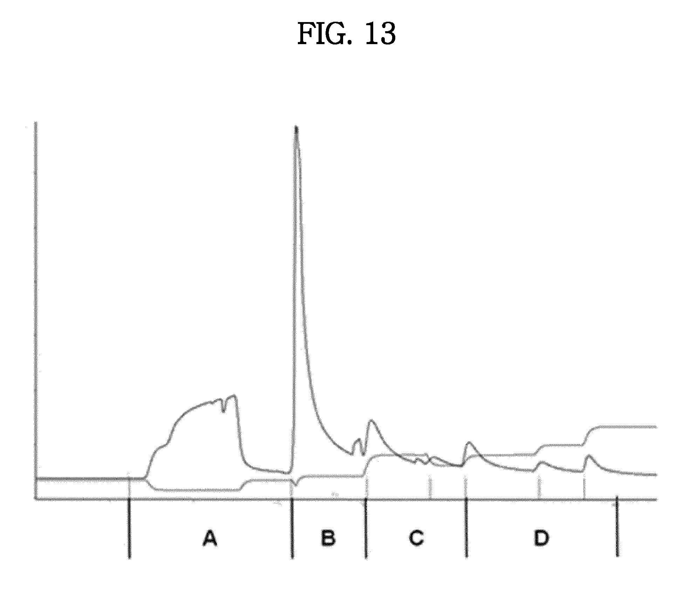

FIG. 13 is a chromatogram showing the light absorbance measured at a UV wavelength of 280 nm for the recombinant FVII fusion protein (FVII-GSI-T-Tf) according to one exemplary embodiment of the present invention after the recombinant FVII fusion protein (FVII-GS1-T-Tf) is isolated and purified by mixed-mode chromatography using XK16/20 ceramic fluoroapatite as a stationary phase.

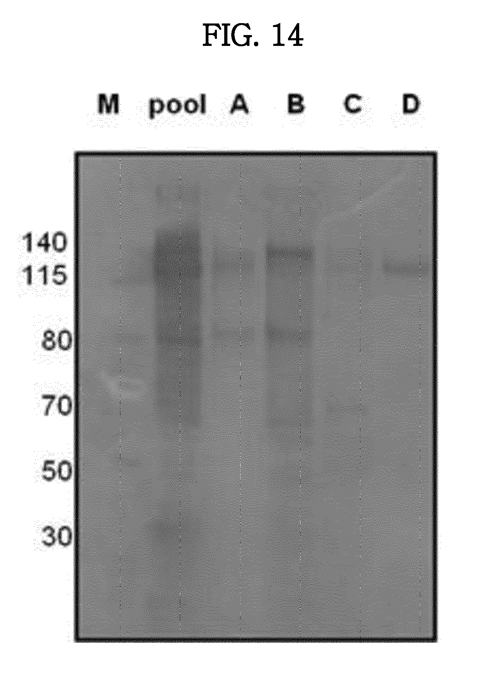

FIG. 14 shows the results analyzed for the recombinant FVII fusion protein (FVII-GS1-T-Tf) according to one exemplary embodiment of the present invention by SDS-PAGE after the recombinant FVII fusion protein (FVII-GS1-T-Tf) is isolated and purified by mixed-mode chromatography using XK16/20 ceramic fluoroapatite as a stationary phase

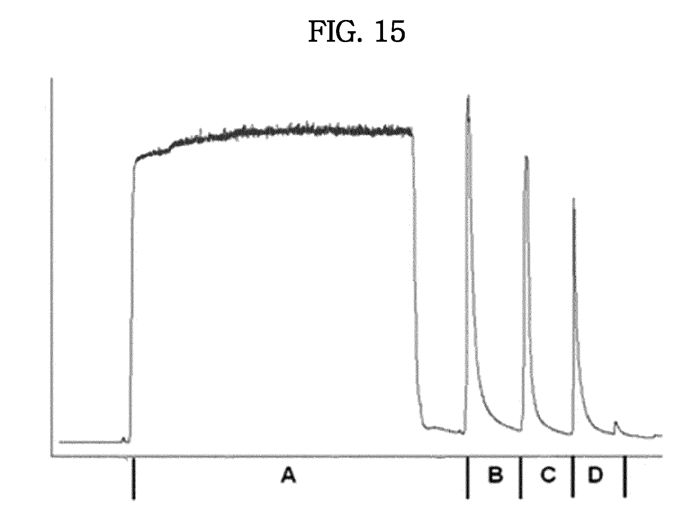

FIG. 15 is a chromatogram showing the light absorbance measured at a UV wavelength of 280 nm for the recombinant FVII fusion protein (FVII-GSI-T-Tf) according to the present invention after the recombinant FVII fusion protein (FVII-GS1-T-Tf) is isolated and purified by mixed-mode chromatography using XK16/20 ceramic hydroxyapatite as a stationary phase.

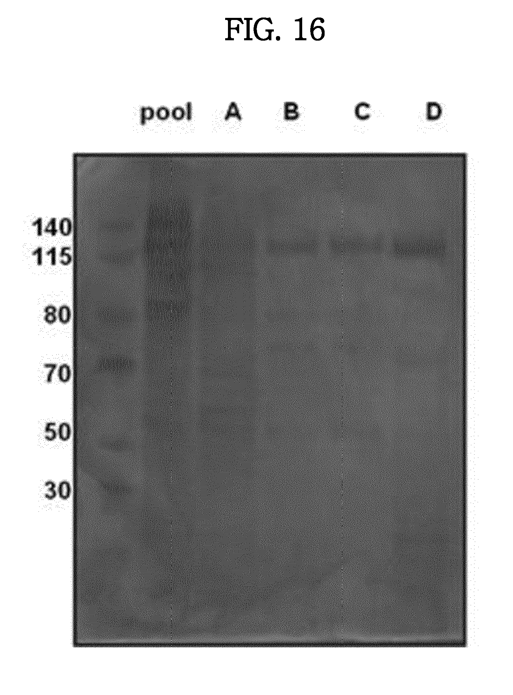

FIG. 16 shows the results analyzed for the recombinant FVII fusion protein (FVII-GS1-T-Tf) according to one exemplary embodiment of the present invention by SDS-PAGE after the recombinant FVII fusion protein (FVII-GS1-T-Tf) is isolated and purified by mixed-mode chromatography using XK16/20 ceramic hydroxyapatite as a stationary phase.

### [Best Mode]

The present invention provides a method of isolating and purifying a fusion protein containing factor VII, which includes 1) isolating and purifying a fusion protein containing factor VII expressed in animal cells by affinity chromatography using a resin having a structure represented by Formula 1 as a stationary phase, or mixed-mode chromatography using a ceramic fluoroapatite gel (Ca₁₀(PO₄)₆F₂) as a stationary phase, and 2) further isolating and purifying the fusion protein by anion exchange chromatography using Q-sepharose FF gel as a stationary phase.

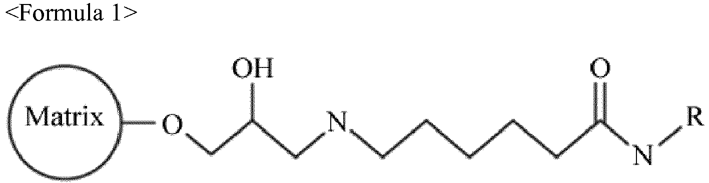

In Formula 1, the matrix is cross-linked agarose, and R is a factor VII binding protein serving as a ligand.

In this specification, the phase "fusion protein containing factor VII" refers to a fusion protein in which blood coagulation factor VII (FVII) is linked to any fusion partner, which is a fusion protein in which the activity of factor VII for blood coagulation, which is not lost or alleviated by the fusion partner.

Examples of the fusion protein includes a fusion protein containing factor VII and transferrin, a fusion protein containing factor VII and albumin, a fusion protein containing factor VII and fibrinogen, a fusion protein containing factor VII and IgA, a fusion protein containing factor VII and IgM, or a fusion protein containing factor VII and a Fc domain of an antibody, but the present invention is not limited thereto.

According to one exemplary embodiment, the fusion protein that may be used in the method of the present invention is a fusion protein containing factor VII (FVII) and transferrin (Tf). The FVII and transferrin residues of the FVII-Tf fusion protein may be derived from any mammal, preferably human FVII and transferrin. More preferably, the FVII residue and transferrin have a sequence homology of 95% or more to respective wild-type proteins found in human blood. Most preferably, FVII has an amino acid sequence set forth in SEQ ID NO: 1, and transferrin has an amino acid sequence set forth in SEQ ID NO: 2.

Also, the FVII-Tf fusion protein includes functional equivalents or functional derivatives having substantially the same functional activities. Examples of such functional equivalents may include variants mutated by any of deletions, insertions, non-conservative or conservative substitutions, or combinations thereof in any amino acid residue in the respective amino acid sequences set forth in SEQ ID NOs: 1 and 2. In this case, such modifications do not substantially alter the active sites or domains which give the biological activities of FVII. Occasionally, the FVII-Tf fusion protein may be altered to enhance or reduce their physical and chemical properties, and it may be modified by phosphorylation, sulfation, acrylation, glycosylation, methylation, farnesylation, acetylation, amidation, etc. As long as the activities of FVII are substantially retained by such modifications, such functional derivatives are also encompassed in the scope of the present invention.

The FVII-Tf fusion protein is preferably a fusion protein in which transferrin is linked to the C-terminus of FVII. Since the N-terminus of FVII is exposed in the fusion protein having FVII and transferrin linked in the order listed herein, the fusion protein has a superior effect as a therapeutic agent, compared to the fusion protein having transferrin and FVII linked in the order listed herein (see Table 3).

Also, the FVII-Tf fusion protein may include a restriction endonuclease recognition sequence between FVII and transferrin in order to promote the insertion of a linker to be described below. Various restriction endonuclease recognition sequences known in the related art may be used as the restriction endonuclease recognition sequence. Preferably, the restriction endonuclease recognition sequence may contain an *Age*I recognition sequence (A/CCGGT). That is, the fusion proteins in which the restriction endonuclease recognition sequence is linked to the C-terminus of FVII and transferrin is linked to the restriction endonuclease recognition sequence is encompassed in the scope of the present invention.

In addition, the FVII-Tf fusion protein may include a linker between the FVII residue and the transferrin residue. The linker may have 1 to 100 amino acids, preferably 1 to 75 amino acids, and more preferably 5 to 25 amino acids. Here, any peptides may be used as long as they can separate the FVII residue and the transferrin residue. The linker may have a stable secondary structure such as a double helix structure, or it may be derived from an IgG hinge region. Preferably, since the linker is freely rotatable in an aqueous solution and does not have a fixed structure, the linker is not immunogenic and minimizes the potential interference of two fusion partners to enhance the FVII activities of the fusion protein. As such a linker, the linker may be a helix linker having an amino acid sequence set forth in SEQ ID NO: 11. Also, such a flexible linker may include glycine (G) and serine (S) in a repeated or random pattern. Examples of the linker include (GGGGS)_{N} (N is an integer ranging from 1 to 20). Preferably, the linker has an amino acid sequence set forth in one of SEQ ID NO: 3 to 10 (see Table 1). Also, an amino acid sequence having homology of 80% or more, preferably having homology of 85% or more to the amino acid sequence of the linker may also be used in the FVII-Tf fusion protein.

Also, the linker may include a protease digestion site recognizable by proteases plentifully present in damaged tissues. The digestion site may be a site cleaved by at least one protease selected from the group consisting of thrombin, factor Xa, factor IXa, and factor VIIa. The fusion protein including such a protease digestion site is cleaved into respective proteins of FVII and transferrin at the working site. Then, the FVII and transferrin serve as the respective proteins. Preferably, the linker has an amino acid sequence set forth in SEQ ID NO: 12 (see Table 1).

The linker may be more readily inserted via the restriction endonuclease recognition sequence inserted between FVII and transferrin. Therefore, the restriction endonuclease recognition sequence(s) may be present at either of upstream or downstream end or both ends of the linker, and it may be translated into amino acids corresponding to the n.t sequence. For example, when an *Age*I restriction endonuclease recognition sequence is used, Thr may be present upstream of the linker, and Thr-Gly may be present downstream of the linker. That is, when a linker such as (GGGGS)₃ is used, the linker is present in the form of -T(GGGGS)₃TG-. An amino acid(s) translated at the upstream or downstream of the linker may vary according to the restriction endonuclease recognition sequence used herein, but the activities of the fusion protein are not changed due to the presence of the translated amino acid(s) (see Table 5).

The FVII-Tf fusion protein has FVII specific activity of 0.7 or more, compared to non-fused wild-type FVII. For example, the fusion protein including the FVII having the amino acid sequence set forth in SEQ ID NO: 1 and the transferrin having the amino acid sequence set forth in SEQ ID NO: 2 has FVII specific activity of approximately 0.82 to 0.92, compared to the non-fused wild-type FVII (see Tables 2 and 3). For example, the fusion protein including the FVII having the amino acid sequence set forth in SEQ ID NO: 1, the linker having the amino acid sequence set forth in SEQ ID NO: 3, and the transferrin having the amino acid sequence set forth in SEQ ID NO: 2 also has FVII specific activity of approximately 0.97, compared to the non-fused wild-type FVII (see Table 2). In addition, a fusion protein having another linker inserted between FVII and transferrin also has FVII specific activity of approximately 0.74 to 1, compared to the non-fused wild-type FVII (see Table 2). Further, the FVII-Tf fusion protein has a half-life 3 to 4 times longer than the FVII not linked with transferrin (see Table 6).

In the present invention, the fusion protein containing factor VII may be expressed by introducing a DNA sequence coding for the fusion protein into host cells.

The DNA sequence coding for the fusion protein may be widely altered or modified within a range in which the amino acid sequence of the fusion protein is not altered, in consideration of the codon degeneracy or codons preferred in organisms in which the fusion protein is expressed. For example, in the case of the FVII-Tf fusion protein, the DNA sequence coding for the FVII-Tf fusion protein is preferably a DNA sequence having the nucleotide sequence set forth in one of SEQ ID NOs: 13 to 24.

The DNA sequence coding for the fusion protein may be introduced into host cells using a recombinant expression vector. In the present invention, the term "vector" refers to a means for introducing a DNA sequence coding for the fusion protein into host cells and expressing the fusion protein, and the term includes all types of conventional vectors including a plasmid vector, a cosmid vector, a bacteriophage vector, and a viral vector, with a plasmid vector being preferred. Proper expression vectors include n.t sequences coding for a signal peptide or a leader peptidee for membrane targeting or secretion as well as expression control elements such as a promoter, an initiation codon, a stop codon, a polyadenylation signal, and an enhancer, and they may be variously prepared according to a purpose of use. The initiation codon and the stop codon should necessarily act in a subject when a gene construct is administered to the subject, and they should be fused in-frame with a coding sequence. Also, the expression vector includes a selectable marker for selecting host cells containing the vector, and a replicable expression vector includes a replication origin. The vector may be self-replicated or integrated into a host cell DNA sequence. Specifically, the recombinant expression vector according to one exemplary embodiment of the present invention may be constructed by inserting a DNA sequence coding for the fusion protein into pcDNA3.1-hygro vector.

In the present invention, conventional animal cells known in the related art may be used as the host cells used to express the fusion protein. Examples of the host cells may include Chinese hamster ovary (CHO) cells, human embryonic kidney cells (HEK293), hamster kidney cells (BHK 21), human liver cancer cells (Hep G2), and the like, but the present invention is not limited thereto.

Methods known in the related art may be used to transform host cells with the recombinant expression vector according to one exemplary embodiment of the present invention. Such methods include electroporation, plasmogamy, calcium phosphate (CaPO₄) precipitation, and calcium chloride (CaCl₂) precipitation, but the present invention is not limited thereto.

The present invention is characterized in that the fusion protein is isolated and purified through the steps of 1) isolating and purifying a fusion protein containing factor VII expressed in animal cells by affinity chromatography using a resin having the above-described structure as a stationary phase, or mixed-mode chromatography using a ceramic fluoroapatite gel (Ca₁₀(PO₄)₆F₂) as a stationary phase, and 2) further isolating and purifying the fusion protein by anion exchange chromatography using a Q-sepharose FF gel as a stationary phase.

Prior to performing the chromatography, the fusion protein according to one exemplary embodiment of the present invention may selectively undergo the following steps:
a) culturing host cells transformed with a recombinant expression vector and isolating the cells from culture medium; and
b) concentrating the cell culture medium by ultrafiltration.

In step a), the host cells are cultured according to culture methods widely known in the related art. Next, the culture is centrifuged to isolate the cells from the culture medium, and the isolated culture medium is applied to step b). The efficiency of ultrafiltration or dialysis in step b) may be enhanced through the centrifugation in step a).

In step b), the isolated cell culture medium may be preferably concentrated by ultrafiltration (UF) using a membrane having a cut-off value (i.e., a retention time) of 30 kDa. The membrane for ultrafiltration may vary according to the characteristics of a target protein. Generally, the target protein may be enriched without any great loss by passing the cell culture medium through a membrane having a smaller pore size than the molecular weight of the target protein. For example, a FVII fusion protein having a molecular weight of 130 kDa may be enriched by passing the cell culture medium through a membrane having a pore size of 30 kDa. After the ultrafiltration, a buffer used in this step may also be replaced with an equilibration buffer used in subsequent procedures. An exemplary equilibration buffer may a buffer (pH 6.5) including 25 mM imidazole and 0.02% Tween 80.

According to one exemplary embodiment, the concentrate having undergone the above the concentration procedure is isolated and purified by affinity chromatography using a resin having a structure represented by the following Formula 1 as a stationary phase.

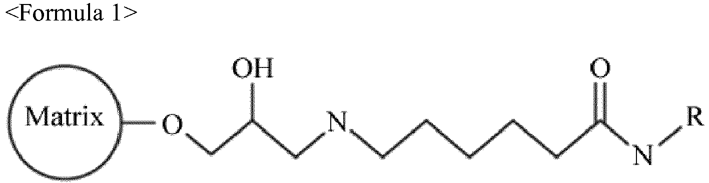

The resin used in the affinity chromatography is composed of a matrix, a hydrophilic cross-linker and a ligand (R). In the structure, the matrix is cross-linked agarose, preferably highly cross-linked high-flow agarose, and the ligand (R) is a *Camelidae*-derived single-domain antibody fragment that is a protein binding to factor VII. The resin may selectively bind to a FVII-derived protein since the protein specifically binding to FVII is linked to an agarose fragment via a covalent bond. Since the ligand also has a long cross-linker showing hydrophilicity, the resin has a structure in which the ligand readily binds to a target protein to be isolated. Examples of the resin may include 'VIISelect' commercially available as a custom-designed medium (GE Healthcare; UK), but the present invention is not limited thereto. An exemplary VIISelect has the following properties.
- Particle size: 75 µm (d₅₀ᵥ)
- Ligand: recombinant protein produced in *Saccharomyces cerevisiae*
- Ligand density: 5.7 mg per 1 mL of medium
- Binding capacity : 8 mg per 1 mL of medium
- Working temperature: 4°C to 30°C

The affinity chromatography using the VIISelect is typically known to elute factor VII with an elution buffer (pH 7.5) including 50 mM Tris, 1.5 M sodium chloride, and 50% propylene glycol. However, the fusion protein including the factor VII according to one exemplary embodiment of the present invention has a problem in that the use of the eluate cause a significant decrease in yield. Therefore, the present invention is technically characterized in that an aqueous sodium thiocyanate (2.5 M) buffer at pH 5.0 to pH 8.0, preferably pH 7.0 is used instead of the conventional known eluates to enhance the isolation and purification of the fusion protein including the factor VII.

According to one exemplary embodiment of the present invention using the affinity chromatography, a resin stationary phase is equilibrated with a buffer (pH 7.5) including 50 mM Tris and 150 mM sodium chloride, and a non-residual fragment is then removed with a washing buffer until the resin stationary phase reaches an equilibrium (which is constantly retained at the baseline). Thereafter, the FVII fusion protein may be isolated and purified using an aqueous sodium thiocyanate (2.5 M) buffer (pH 7.0).

The recovery capacity of the FVII fusion protein may be determined by measuring the light absorbance at a wavelength of 280 nm. The activity of the recovered protein may be determined by measuring the amount of light scattered when red light (660 nm) passes through a mixed solution of sample plasma and a reagent, which indicates, a degree of blood coagulation, using a blood coagulation analyzer. According to one exemplary embodiment of the present invention, the FVII fusion protein having a purity of 95% or more may be recovered in a yield of 90% or more using the above-described affinity chromatography.

According to another exemplary embodiment, the FVII fusion protein of the present invention may be isolated and purified by mixed-mode chromatography using a ceramic fluoroapatite gel (Ca₁₀(PO₄)₆F₂) as a stationary phase.

A column using the ceramic fluoroapatite gel as the stationary phase is a column used to isolate proteins having a range of low pI values based on charge interaction. The column has a structure similar to the commercially available ceramic hydroxyapatite column manufactured by Novo. The ceramic fluoroapatite has a structure in which a hydroxyl (OH⁻) group of the ceramic hydroxyapatite is replaced with fluorine (F⁻). the fluorine ions are known to increase the stability of the resin at an acidic condition, compared to the hydroxyl groups.

The stationary phase of ceramic fluoroapatite is equilibrated with a buffer (pH 6.5) including 25 mM imidazole and 0.02% Tween 80. A non-residual fragment is removed with a washing buffer until the stationary phase reaches an equilibrium (which is constantly retained at the baseline). As the elution buffer to elute or for elution of the factor FVII, an aqueous buffer (pH 5.0 to 8.0) including sodium phosphate and sodium chloride, preferably an elution buffer (pH 6.3) including 25 mM imidazole, 0.02% Tween 80, and 180 to 340 mM Na-Pi may be used. The recovery capacity of the FVII fusion protein may be determined by measuring the light absorbance at a wavelength of 280 nm. The activity of the recovered protein may be determined by analyzing the chromogenic activity using COASET FVII (Chromogenix, #821900-63) analytic kit. In the present invention, the FVII fusion protein having a purity of 95% or more may be recovered in a yield of 90% or more by performing the anion exchange chromatography.

In the present invention, the fusion protein undergoing the affinity chromatography or the mixed-mode chromatography may be further isolated and purified with anion exchange chromatography using Q-sepharose FF gel as a stationary phase.

The Q-sepharose FF gel is a potent anion exchanger. In this case, when a net charge has a negative value at a certain pH, an amine group of a protein may bind to an amine exchanger through a charge exchange to remove a low level of non-specifically binding impurities of the host cells.

The stationary phase of the Q-sepharose FF gel is equilibrated with a buffer (pH 8.0) including 20 mM Tris, 20 mM sodium chloride, and 5 mM calcium chloride. A non-residual fragment is removed with a washing buffer until the stationary phase reaches an equilibrium (which is constantly retained at the baseline). As the elution buffer for elution of the activated factor FVII fusion protein, an aqueous buffer (pH 5.0 to 8.0) including 10 mM to 50 mM Tris, 1 mM to 10 mM calcium chloride, and 50 mM to 150 mM sodium chloride, preferably an aqueous buffer (pH 5.0 to 8.0) including 20 to 40 mM Tris, 2 to 5 mM calcium chloride, and 60 to 100 mM sodium chloride, and more preferably a buffer (pH 8.0) including 20 mM Tris, 5 mM calcium chloride, and 86.64 mM sodium chloride may be used.

The recovery capacity of the FVII fusion protein may be determined by measuring the light absorbance at a wavelength of 280 nm. The activity of the recovered protein may be determined by analyzing the chromogenic activity using COASET FVII (Chromogenix, #821900-63) analytic kit. The FVII fusion protein having a purity of 95% or more may be recovered in a yield of 50% or more by performing the anion exchange chromatography.

According to one exemplary embodiment of the present invention, the FVII fusion protein having a purity of 95% or more may be recovered from a cell culture medium in a yield of 50% or more using affinity chromatography using VIISelect or mixed-mode chromatography using ceramic fluoroapatite and anion exchange chromatography using a Q-sepharose FF gel.

### [Mode for Invention]

Hereinafter, the present invention will be described in further detail with reference to the following exemplary embodiments. However, it should be understood that the description proposed herein is just a preferable example for the purpose of illustrations only, not intended to limit the scope of the invention.

### Example 1: Construction of factor VII (FVII) plasmid vector (pcDNA3.1-hygro-FVII)

RNA purified from Hep G2 cells (KCLB No. 88065) was used as a template for reverse transcription. A cDNA transcript was amplified by PCR using FVII gene specific primers, FVII-F and FVII-R (SEQ ID NOs: 25 and 26) to obtain an open reading frame (ORF) of a human FVII gene. The PCR was as follows: 50 µL of a reaction solution (including 0.5 µL of cDNA, 0.4 µM of respective primers (10 pmol/µL) set forth in SEQ ID NOs: 25 and 26, 0.2 mM dNTP, 5 units of Taq DNA polymerase, and water) was reacted at 94°C for 5 minutes; reacted for 35 cycles, with one cycle consisting of denaturation at 94°C for 1 minute, annealing at 60°C for 1 minute, and extension at 72°C for 2.5 minutes; and then reacted at 72°C for 5 minutes to terminate the reaction. The purified PCR product was cloned into a pGEM-T easy vector (Promega, Cat No.: A1360). The cloned vector was treated with restriction endonucleases *EcoR*I and *Nco*I to screen positive clones. The screened clones were verified by DNA sequencing. To transfer an ORF of the FVII (FVII-ORF) to an expression vector, FVII-ORF of a pGEM-T easy vector was digested with a restriction endonuclease *Not*I. The FVII-ORF digested with NotI was treated with T4 DNA polymerase to make blunt ends and ligated to a pcDNA3.1-hygro vector (Invitrogen) treated with restriction endonucleases *Hind*III/*Xba*I*.* The ligated of vector and FVII-ORF fragment were verified by treatment with restriction endonucleases *Apa*I, *Xba*I, *EcoR*I, *Nco*I, and *Pst*I and by DNA sequencing. This vector was named "pcDNA3.1-hygro-FVII."

### Example 2: Construction of FVII-Tf expression vector (pcDNA3.1-hygro-FVII-TF)

The FVII cDNA prepared in Example 1 was connented to human transferrin (Tf) cDNA and expressed as a single zymogen in animal cells. Human transferrin cDNA was purchased from Origene (Cat No.: SC322130) to obtain cDNA having the same sequence as GenBank Accession No: NM_001063.2. Primers used for ligation were designed to remove a stop codon of FVII and nucleotides coding for a signal peptide of Tf. Thereafter, to insert linkers having various sizes between FVII and Tf, an *Age*I site (ACCGGT) translated into threonine (Thr) and glycine (Gly) was added to a linking primer. A fusion protein has a structure of (leader peptide)-(mature FVII)-(Thr-Gly)-(mature Tf) (the leader peptide includes a combination of a signal peptide (a prepeptide) not present in mature FVII and a peptide (a propeptide) cleaved by a processing enzyme, consists of 38 amino acids and corresponds to the amino acids at the 1^{st} to 38^{th} positions in the amino acid sequence set forth in SEQ ID NO: 1). The cDNAs of FVII and Tf were amplified using the primers FVII-S1, FVII-AS1, Tf-S1, and Tf-AS1 (SEQ ID NOs: 27 to 30), and the vectors described in Example 1 were used as the vector. The primers set forth in SEQ ID NOs: 27 and 30 include NheI and XhoI sites, respectively. A cloning scheme in which FVII cDNA is ligated to Tf cDNA is shown in FIG. 1. First, FVII cDNA was amplified from the pcDNA3.1-hygro-FVII vector prepared in Example 1 using PCR. The PCR was as follows: 50 µL of a reaction solution (including 1 µL of a mixed vector template, 1 µL of primers FVII-S1 and FVII-AS1 (10 µM each), 10 µL of a 5 × Phusion HF buffer, 200 µM dNTP, 0.5 µL of a Phusion DNA polymerase (FINNZYMES, #F-530S, 2 units/µL), and 35.5 µL of water) was reacted at 98°C for 30 seconds; reacted for 30 cycles, with one cycle consisting of denaturation at 98°C for 10 seconds, annealing at 60°C for 45 seconds, and extension at 72°C for 30 seconds; and then reacted at 72°C for 7 minute to terminate the reaction.

Next, Tf was amplified using human transferrin cDNA as a template. The reaction was performed in the same manner as in the FVII PCR conditions, except that the primers Tf-S1 (10 µM) and Tf-AS1 (10 µM) were used.

The amplified FVII and Tf cDNAs were linked through a series of restriction endonuclease digestions and ligations. Each of the cDNA fragments amplified by PCR was treated with the restriction endonucleases *Age*I and *Xho*I, or *Nhe*I. The cDNA fragments of the FVII and Tf treated with the restriction endonuclease(s) was purified and ligated at a molar ratio of 1:1. The ligated DNA was sub-cloned into pcDNA3.1-hygro vector (Invitrogen) treated with the restriction endonucleases *Nhe*I/*Sho*I*.* The size and sequence of the insert fragment were verified by DNA sequencing.

### Example 3: Construction of FVII-GS linker-Tf expression vector

A peptide consisting of 5 amino acids including glycine and serine was used as a basic linker unit. The basic linker unit includes four glycine residues and one serine residue such as a 'GGGGS.' The basic GS linker unit (hereinafter referred to as "GS-X linker" where X represents the number of repeats of the basic GS linker unit) was used to construct longer GS linkers. In the present invention, linkers ranging from GS-1 to GS-15 were constructed.

### 1) Construction of FVII-GS-1 linker-Tf expression vector

Primers GS-FV-AS1 and GS-Tf-S1 (SEQ ID NOs: 31 and 32) including a sequence of the basic GS linker unit were synthesized, and a GS-1 linker was inserted between FVII and Tf by overlapping PCR (see FIG. 2).

PCR was performed with Phusion DNA polymerase (FINNZYMES, #F-530S) using the primers FVII-S1 and GS-FV-AS1 (SEQ ID NOs: 27 and 31) to ligate the GS-1 linker to FVII. The PCR was as follows: 50 µL of a reaction solution (including 1 µL of a pcDNA3.1-hygro-FVII-Tf vector, 1 µL of FVII-S1 (10 pmole/µL), 1 µL of GS-FV-AS1 (10 pmole/µL), 1 µL of 10 mM dNTP, 10 µL of a 5× Phusion HF buffer, 35.5 µL of water, 0.5 µL of Phusion DNA polymerase (2 unit/µL)) was reacted at 98°C for 30 seconds; reacted for 30 cycles, with one cycle consisting of denaturation at 98°C for 10 seconds, annealing at 64°C for 30 seconds, and extension at 72°C for 45 seconds; and then reacted at 72°C for 7 minute to terminate the reaction. Meanwhile, the PCR was performed in the same manner, except that the primers (SEQ ID NO: 32 and 30) for GS-Tf-S1 and Tf-AS1 were used instead of the primers in the reaction solution to ligate Tf to the GS-1 linker. Overlapping PCR was performed using the amplified PCR products as templates. The overlapping PCR was as follows: a reaction solution including 1 µL of each of the amplified PCR products, 1 µL of FVII-S1 (10 pmole/µL, SEQ ID NO: 27), 1 µL of an antisense primer (Tf-AS1 10 pmole/µL, SEQ ID NO: 30), 10 µL of a 5× Phusion HF buffer, 1 µL of 10 mM dNTP, 34.5 µL of water, and 0.5 µL of Phusion DNA polymerase (2 units/µL) was reacted at 98°C for 1 minute; reacted for 45 cycles, with one cycle consisting of denaturation at 98°C for 10 seconds, annealing at 66/68°C for 30 seconds, and extension at 72°C for 45 seconds; and then reacted at 72°C for 7 minute to terminate the reaction. The amplified overlapping PCR product was digested with the restriction endonucleases *Nhe*I and *Xho*I and cloned into a pcDNA3.1-hygro-lacZ vector.

### 2) Construction of FVII-GS-3 linker-Tf expression vector

Primers GS3-S and GS3-AS (SEQ ID NOs: 33 and 34) including GS-3 and *Age*I sites were synthesized. To prepare a GS-3 double helix linker, 5 µL of GS3-S (100 pmole/µL), 5 µL of GS3-AS (100 pmole/µL), 2 µL of a 10× annealing buffer (100 mM Tris-Cl [pH 8.0], 1 M NaCl, and 10 mM EDTA), and 8 µL of water were mixed, heated at 98°C for 10 minutes, and then cooled at 25°C for 1 hour to perform annealing. The annealed linker was digested with the restriction endonuclease *Age*I, and the pcDNA3.1-hygro-FVII-Tf vector prepared in Example 2 was also digested with *Age*I. The digested vector was treated with 1 µL of calf intestinal phosphatase (CIP; NEB, #M0290S) at 37°C for 1 hour, subjected to a gel extraction procedure (QIAGEN, #28704), and ligated with the annealed linker at a molar ratio of 1:3 (vector:insert) using a T4 DNA ligase (TAKARA, #2011A).

### 3) Construction of FVII-GS-5 linker-Tf expression vector and FVII-GS-15 linker-Tf expression vector

A new method was introduced to construct a fusion protein expression vector including a GS-5 linker. A FVII-Tf fusion vector including a linker extended through the following two steps was constructed.

The first step is to add a synthesized double-stranded (ds) GS-2 linker to the GS-3 linker obtained above. After the extension of the linker was confirmed, the linker was digested and inserted between FVII and Tf genes of the pcDNA3.1-hygro-FVII-Tf vector. To extend the GS-3 linker to a GS-5 linker, for example, a synthesized dsGS-2 unit set forth in SEQ ID NO: 35 was treated with *Bgl*II and then ligated to a pcDNA3.1-hygro-FVII-GS-3-Tf vector treated with the restriction endonucleases *Bam*HI and *Stu*I*.* Subsequently, the extension of the linker was verified through the treatment with *Bam*HI and *Age*I*,* and the extended linker was then digested with *Age*I*,* and sub-cloned into a pcDNA3.1-hygro-FVII-Tf vector treated with *Age*I and CIP. FVII-Tf fusion expression vectors including the GS-7, GS-9, GS-11, GS-13, and GS-15 linkers were also constructed in the same manner (see FIG. 3).

### Example 4: Construction of FVII-Tf expression vector (pcDNA3.1-hygro-FVII-GS1-T-Tf) including linker containing thrombin restriction site

Thrombin cleavage sites were linked to both ends of the GS-1 unit (hereinafter referred to as a "GS1-T linker"). A dsGS1-T linker set forth in SEQ ID NO: 36 (sense) was synthesized so that both ends of the dsGS1-T linker had *Age*I sites. The dsGS1-T linker was treated with *Age*I and purified using a PCR purification kit (Qiagen, Cat. No.: 28104). The purified linker was ligated to pcDNA3.1-hygro-FVII-Tf vector treated with CIP/*Age*I*.*

### Example 5: Construction of FVII-Tf expression vector (pcDNA3.1-hygro-FVII-Helix-Tf) including helix linker

A helix linker DNA was prepared using the method disclosed in US Patent Application No: 2009/0170163. *Age*I sites were added to both ends of the prepared helix linker DNA using the primers Helix linker S and Helix linker AS (SEQ ID NOs: 37 and 38). A helix linker obtained by annealing the primers Helix linker S and Helix linker AS (SEQ ID NOs: 37 and 38) was treated with *Age*I and then inserted into a pcDNA3.1-hygro-FVII-Tf vector treated with *Age*I and CIP. The prepared pcDNA-hygro-FVII-Helix-Tf vector was verified by DNA sequencing.

The characteristics of the expression vectors constructed in Examples 2 to 5 are listed in the following Table 1.

**[Table 1]**

| FVII fusion protein | C-terminus of FVII | Linker sequence (sequence identification number) | N-terminus of fusion partner | Number of amino acids in linker |
|---|---|---|---|---|
| FVII-Tf | APFP | - | VPDKTV | 0 |
| FVII-GS1-Tf | APFP | GGGGS (SEQ ID NO: 3) | VPDKTV | 5 |
| FVII-GS3-Tf | APFP | (GGGGS)₃ (SEQ ID NO: 4) | VPDKTV | 15 |
| FVII-GSS-Tf | APFP | (GGGGS)₅ (SEQ ID NO: 5) | VPDKTV | 25 |
| FVII-GS7-Tf | APFP | (GGGGS)₇ (SEQ ID NO: 6) | VPDKTV | 35 |
| FVII-GS9-Tf | APFP | (GGGGS)₉ (SEQ ID NO: 7) | VPDKTV | 45 |
| FVII-GS11-Tf | APFP | (GGGGS)₁₁ (SEQ ID NO: 8) | VPDKTV | 55 |
| FVII-GS13-Tf | APFP | (GGGGS)₁₃ (SEQ ID NO: 9) | VPDKTV | 65 |
| FVII-GS15-Tf | APFP | (GGGGS)₁₅ (SEQ ID NO: 10) | VPDKTV | 75 |
| FVII-GS1-T-Tf | APFP | GGGGSLVPRGSGGGS (SEQ ID NO: 12) | VPDKTV | 15 |
| FVII-Helix-Tf | APFP | GA(EAAAK)₄A (SEQ ID NO: 11) | VPDKTV | 23 |
| * Thr-Gly derived from *Age*I is present in function protein FVII-Tf. | | | | |
| * Thr derived from *Age*I is present upstream of the linker sequences set forth in SEQ ID NOs: 4 to 12, and Thr-Gly derived from *Age*I is present downstream of the linker sequences. | | | | |

### Experimental Example 1: Measurement of specific activity of FVII-fusion protein

The FVII-fusion proteins prepared in Examples 2 to 5 were expressed in CHO (VK2) cells that are derived from the CHO cell line in which vitamin K epoxide reductase complex subunit 1 (VKORC1) is stably expressed.

The expression vectors constructed in Examples 2 to 5 were purified using Endo-free plasmid maxi kit (Qiagen, #27104). β-Galactosidase was used as the control for transfection. The CHO (VK2) cells were seeded in a 6-well plate at a concentration of 1.5×10⁶/well. The cells were cultured for 24 hours in α-MEM (Lonza, #12-169F) supplemented with 10% FBS (Lonza, #14-501F), 1× HT (Invitrogen, #11067-030), 4 mM L-glutamine (Lonza, #17-605E), and 200 µg/mL of hygromycin (Invitrogen, #10687-010), and the cells were transfected with Lipofectamine 2000 (Invitrogen) according to the supplier's manual. After 4 hours of transfection, the culture medium was replaced with a serum-free culture medium (OptiMEM), and 5 µg/mL of vitamin K was added thereto. After 48 hours of the culture, the culture medium was sampled and stored at -70°C.

The chromogenic activities of the expressed FVII-fusion proteins and the amounts of the antigens were analyzed using COATEST Factor VII analytic kit (Chrmogenix, #821900-63) and a FVII ELISA kit (Cedarlene Lab, #CL20030K), respectively. The analysis was performed according to the manufacturer's manual. The standard human plasma standardized for the WHO international standard was used as the control FVII for quantitative analysis. The expression of proteins was confirmed by Western blotting technique using FVII and Tf antibodies. Based on the ELISA results, that the expressed FVII-fusion proteins had an expected size without any fragmentation when the same amount of the FVII fusion proteins was loaded (see FIG. 4).

Meanwhile, the specific activities of the FVII-transferrin fusion proteins were in a range of 0.74 to 1 (see Table 2). The FVII-transferrin fusion proteins including the linker also had FVII activity of 70% or more. The length of the linker and the specific activities of the FVII fusion proteins were shown to have no certain relationship, but the FVII fusion proteins including a shorter linker had a slightly higher specific activity than the FVII fusion proteins including a longer linker. Especially, the FVII-GS1-Tf and FVII-GS1-T-Tf fusion proteins had a remarkably high specific activity (see FIG. 5).

**[Table 2]**

| FVII fusion protein | Antigen (%) | Activity (%) | Specific activity (activity/antigen) |
|---|---|---|---|
| FVII-Tf | 53.2 ± 5.0 | 43.9 ± 0.3 | 0.82 |
| FVII-GS1-Tf | 53.4 ± 3.1 | 52.0 ± 0.5 | 0.97 |
| FVII-GS3-Tf | 61.9 ± 8.0 | 57.7 ± 0.2 | 0.93 |
| FVII-GS5-Tf | 69.3 ± 5.6 | 55.9 ± 1.4 | 0.81 |
| FVII-GS7-Tf | 70.9 ± 8.2 | 59.3 ± 1.1 | 0.84 |
| FVII-GS9-Tf | 64.2 ± 8.6 | 47.5 ± 0.7 | 0.74 |
| FVII-GS11-Tf | 59.1 ± 3.9 | 45.3 ± 0.9 | 0.77 |
| FVII-GS13-Tf | 59.7 ± 5.1 | 49.1 ± 0.8 | 0.82 |
| FVII-GS15-Tf | 59.2 ± 6.0 | 50.2 ± 0.5 | 0.85 |
| FVII-GS1-T-Tf | 70.8 ± 8.7 | 71.0 ± 2.6 | 1.00 |
| FVII-Helix-Tf | 89.0 ± 5.7 | 78.9 ± 2.2 | 0.89 |

### Example 6: Characterization of FVII fusion protein according to Tf fusion direction

To observe the differences in characteristics of the fusion proteins according to the fusion direction, a fusion protein in which human transferrin (Tf) was linked to the N-terminus of FVII was prepared, and its activity was compared to a fusion protein in which human transferrin (Tf) was linked to the C-terminus of FVII. Specific procedures were as follows.

### <6-1> Construction of Tf FVII and Tf-GS1T - FVII expression vectors

Two fusion proteins in which Tf was linked to the N-terminus of FVII were prepared. In this case, the fusion proteins had the following structures: 1) (leader peptide of Tf)-(mature Tf)-(Thr-Gly)-(mature FVII); and 2) (leader peptide of Tf)-(mature Tf)-(Thr)-(GS1-T; SEQ ID NO: 12)-(Thr-Gly)-(mature FVII).

To obtain a gene sequence of Tf including a coding n.t sequence for the leader peptide, first, a forward primer (Nhe-Tf: SEQ ID NO: 46) used for amplification was designed to include a *Nhe*I site for the purpose of cloning, and a reverse primer (Tf-Age: SEQ ID NO: 47) was designed to remove a stop codon of Tf and to include a *Nhe*I site for the purpose of cloning as well. A forward primer (Age-FVII: SEQ ID NO: 48) used to clone mature FVII from which the leader peptide was removed was designed to include a restriction endonuclease *Age*I, and a reverse primer (VII-Xho: SEQ ID NO: 49) was designed to include a restriction endonuclease *Xho*I.

In the case of the Tf gene, cDNA purchased from Origene (Cat No.: SC322130) was used as a PCR template as described in Example 2. The PCR was performed as follows: 50 µL of a reaction solution (including 1 µL of vector template, 2 µL of primers Nhe-Tf and Tf*-Age*I (10 µM for each), 10 µL of a 5× Phusion HF buffer, 1 µL of 10 mM dNTP, 0.5 µL of Phusion DNA polymerase (FINNZYMES, #F-530S, 2 units/ µL), and 33.5 µL of water) was reacted at 98°C for 30 seconds; reacted for 25 cycles, with one cycle consisting of denaturation at 98°C for 10 seconds, annealing at 70°C for 30 seconds, and extension at 72°C for 36 seconds; and then reacted at 72°C for 10 minute to terminate the reaction. FVII was amplified by PCR using the pcDNA3.1-hygro-FVII-GS1-T-Tf vector constructed in Example 4 as the template. The reaction was performed in the same manner as in the Tf PCR conditions, except that the primers Age-FVII (10 µM) and VII-Xho (10 µM) were used.

The Tf gene amplified by the PCR was digested with the *Nhe*I/*Age*I restriction endonucleases, and the digested fragment of the Tf PCR product was inserted into a pcDNA3.1-hygro-FVII-GS1T-Tf vector to obtain a pcDNA3.1-hygro-Tf-Tf vector. The obtained pcDNA3.1-hygro-Tf-Tf vector and the FVII PCR products were treated with the *Age*I/*Xho*I restriction endonucleases, and then the vector and the FVII PCR product were ligated to construct an expression vector including the pcDNA3.1-hygro-Tf-FVII fusion protein. The pcDNA3.1-hygro-Tf-GS1-T-FVII expression vector was constructed by treating the dsGS1-T sequence synthesized as in Example 4 with the *Age*I restriction endonuclease and by inserting the dsGS1-T sequence into the obtained pcDNA3.1-hygro-Tf-FVII. The restriction endonuclease mapping and nucleotide sequencing confirmed that the obtained expression vectors were constructed as intended.

### <6-2> Expression and characterization of fusion protein

To characterize the proteins in which Tf was fused to the C-terminus of FVII and the proteins in which Tf was fused to the N-terminus of FVII, each of the expression vectors (pcDNA3.1-hygro-FVII-Tf, pcDNA3.1-hygro-FVII-GS1T-FVII, pcDNA3.1-hygro-Tf-FVII, and pcDNA3.1-hygro-Tf-GS1-T-FVII) was temporarily expressed in CHO cells.

The 4 constructed plasmid DNAs were isolated using Endo-free maxi prep kit (Qiagen). Before a day of transfection, the CHO (DG44) cells cultured in a T75 flask were treated with trypsin to be detached from the T75 flask, and then seeded in a 6-well plate at a concentration of 1.5×10⁶ cell/well. After 24 hours, the cells were transfected according to the manufacturer's manual. After 4 hours of transfection, the medium was removed from each well, and replaced with 2 mL of a growth medium supplemented with 5 µg/mL of vitamin K. After the transfection, the cells in the 6-well plate were cultured at 37°C in a 5% CO₂ incubator. After 48 hours, the culture medium was centrifuged to obtain supernatant. Then, the supernatant was divided into 1.5 mL tubes, stored at -70°C, and used for a FVII chromogenic assay and FVII ELISA. The medium-free plate was washed with 2 mL of HBSS per a well, and 250 µL of a lysis solution (Tropix, #ABX210LM, supplemented with 1 mM DTT) was evenly spread on the plate and then stored at -70°C for β-galactosidase analysis.

The FVII chromogenic assay and FVII ELISA were performed in the same manner as in Experimental Example 1. A medium stored in a freezer after the transfection was thawed just before the experiment and centrifuged to obtain a supernatant. Then, the supernatant was used as a sample to be analyzed. Standard human plasma (Dade Behring, # ORKL13, Lot#503216F) was used as the standard product for the analysi.

The measurement results are listed in the following Table 3. In the case of the Tf-FVII and Tf-GS1T-Tf fusion proteins in which Tf was linked to the N-terminus of FVII, no activity was measured, unlike the FVII-Tf and FVII-GS1T-Tf fusion proteins in which Tf was linked to the C-terminus of FVII. Also, when the amount of the fusion proteins was measured by FVII ELISA, the amount of the fusion proteins in which Tf was linked to the N-terminus of FVII was detected at a low level. However, when the fusion proteins were subjected to Western blotting using polyclonal antibodies against Tf, the fusion proteins were detected with similar detection sensitivity, regardless of the fusion direction. The results showed that, when Tf is fused to the N-terminus of FVII, the fusion proteins was normally translated, but the fusion protein had no FVII activity.

**[Table 3]**

| Fusion proteins | FVII activity (%) | FVII antigen (%) | Specific activity (activity/antigen) |
|---|---|---|---|
| FVII-Tf | 33.8 ± 0.73 | 37.0 ± 2.17 | 0.92 |
| Tf-FVII | Activity not measurable | 8.0 ± 1.51 | - |
| FVII-GS-1-T-Tf | 46.6 ± 0.29 | 43.0 ± 4.75 | 1.08 |
| Tf-GS-1-T-Tf | Activity not measurable | 13.5 ± 1.01 | - |

### Example 7: Characterization of fusion protein according to the removal of restriction endonuclease recognition sequence used for fusion

To promote the insertion of various linkers between FVII and Tf upon fusion of FVII and Tf in Example 2, a restriction endonuclease (*Age*I) recognition sequence was used. As a result, some fusion proteins had Thr and Gly residues encoded by the restriction endonuclease at both ends of the linker. Accordingly, in this example, it was checked whether the properties of the fusion proteins were changed according to the presence of the restriction endonuclease (*Age*I) recognition sequence.

In this example, the FVII-GS1-T-Tf fusion protein including the GS1-T linker were subjected to PCR-based site-directed mutagenesis, using a mutagenic primer, to remove the restriction endonuclease recognition sequence. As shown in FIG. 6, the Thr residue upstream of the linker and the Thr-Gly residues downstream of the linker were removed. In this case, the primers used are listed in the following Table 4.

**[Table 4]**

| Kinds of primers | Sequences (5'->3') | SEQ ID NOs |
|---|---|---|
| TG del-S | CAG CGG AGG CGG TTC AGT CCC TGA TAA AAC TG | 50 |
| TG del-AS | CAG TTT TAT CAG GGA CTG AAC CGC CTC CGC TG | 51 |
| T del-S | CGA GCC CCA TTT CCC GGT GGA GGC GGA TC | 52 |
| T del-AS | GAT CCG CCT CCA CCG GGA AAT GGG GCT CG | 53 |

### <7-1> Thr-Gly Removal

Mutagenesis was performed using a PCR method. The PCR conditions was as follows: 1 µL of pcDNA3.1-hygro-FVII-GS1T-Tf vector, 0.2 µL of a sense primer (TG del-S 10 µM), 0.2 µL of an antisense primer (TG del-AS 10 µM), 1 µL of 10 mM dNTP, 4 µL of a 5× PCR buffer, 14 µL of distilled water, and 0.2 µL of Phusion DNA polymerase (FINNZYMES, #F-530S) were added and reacted at 98°C for 30 seconds (one cycle); reacted for 18 cycles, with one cycle consisting of denaturation at 98°C for 10 seconds, annealing at 58°C for 30 seconds, and extension at 72°C for 3 minutes; and then reacted at 72°C for 7 minute (one cycle). To remove the original template DNA, 1 µL of *Dpn*I (NEB, #R0176S) was added to the previously amplified PCR products, and the resulting reaction solution was then incuvated at 37°C for 1 hour. 50 µL of HIT competent cells (DH5α, RH617) was transformed with 10 µL of the DNA treated with *Dpn*I and cultured overnight in an LB+amp (10 mg/mL) solid medium. The N.T sequences of the 4 clones obtained from the transformation were analyzed. Among them, the two clones were confirmed to be mutated.

### <7-2> Thr Removal

Mutagenesis was performed in the same manner as in Example <7-1> except using different kinds of primers. That is, PCR-based mutagenesis was performed under the same condition using 1 µL of plasmid DNA in the clone whose mutation was confirmed in Example <7-1> as the template and using 1 µL of a sense primer (T del-S 10 pmole) and 1 µL of an antisense primer (T del-AS 10pmole). The 4 selected clones were subjected to nucleotide sequencing. Among these, the 3 clones were confirmed to be mutated. The resulting expression vector was named "pcDNA3.1-hygro-FVII-GS1-T-Tf(M3)."

### <7-3> Characterization of fusion protein from which restriction endonuclease recognition sequence is removed

CHO cells were transfected with the FVII-GS1-T-Tf and the FVII-GS1-T-Tf(M3) expression vectors to obtain medium supernatants. The obtained supernatants were subjected to an FVII chromogenic assay (Chromogenix) and FVII ELISA (Cedarlane) to determine a change in activity/antigen ratio. As a result, the amount of the antigens and the activities of the FVII-GS1T-Tf and FVII-GS1T-Tf(M3) fusion proteins were almost identical, and there was no change in activity/antigen ratio (specific activity), as listed in Table 5.

**[Table 5]**

| | FVII antigen (%) | FVII activity (%) | Specific activity (activity/antigen) |
|---|---|---|---|
| FVII-GS1-T-Tf | 34.1 ± 2.1 | 39.6 ± 2.2 | 1.16 |
| FVII-GS1-T-Tf(M3) | 33.5 ± 4.7 | 38.0 ± 0.7 | 1.14 |

### Example 8: Measurement of half-life of the fusion protein

This example was performed to check an effect of improving the half-lives of the fusion proteins according to one exemplary embodiment of the present invention. The FVII-Tf, FVII-GS1-Tf, FVII-GS3-Tf, FVII-GS15-Tf, and FVII-GS1-T-Tf fusion proteins were used as the fusion proteins used in this example, and wild-type FVII and commercially available FVIIa (NovoSeven^{®}, Novo Nordisk), were used as non-fusion protein as the coltrols.

### <8-1> Sample preparation

### 1) Preparation of expression medium

Five expression vectors for FVII fusion proteins in which FVII and Tf were fused with each other and an expression vector for FVII which is not a fusion protein were expressed in a FreeStyle CHO-S cell line (Invitrogen, Cat. No.: R800-07). The CHO-S cells were suspended and cultured in a spinner flask containing a FreeStyle CHO expression medium supplemented with 8 mM L-Glu (GIBCO, L-glutamine 200 mM (100×), Cat. No.: 25030-081). The cultured cells were seeded at a density of 4×10⁵ cells/mL before 24 hours of transformation, and the cells were transfected when the density of the cells had reached 1×10⁶ cells/mL. The DNA used for transfection was prepared using Endo-free maxi prep kit (QIAGEN, Cat No.: 12362) or Endo-free plasmid prep kit (QIAGEN, Cat No.:12381), and the transfection was performed according to the transfection protocol with FreeStyle MAX reagent (Invitrogen, Cat No.: 16447-100) transfection protocol. 500 µg of DNA was added to 8 mL of OptiPRO SFM (Invitrogen, Cat No.: 12309-019) and then mixed. Also, 8 mL of OptiPRO SFM (Invitrogen, Cat No.: 12309-019) and 500 µL of a FreeStyle MAX reagent were added into another tube, and OptPRO SFM and FreeStyle MAX reagent were carefully mixed, and kept at room temperature for 10 minutes. After 10 minutes, FreeStyle CHO-S cells were transfected with each of the mixtures. The transfected cells were cultured at 37°C for 3 to 5 days in a 5% CO₂ incubator, and the supernatants were fractionated.

### 2) Purification of the expression medium

To remove the cells and cell debris remaining in the culture medium obtained by the spinner flask culture, the culture medium was filtered through a 0.22 µm filter (Corning). The filtered culture medium was 10 times concentrated by ultrafiltration using a tangential-flow membrane (Satorious, 30 kDa). The concentrated medium was applied to an XK16/20 (GE healthcare) column packed with ceramic hydroxyapatite (BIO-RAD, 157-0040) resin. The hydroxyapatite column was equilibrated with 10× column volume of an equilibration buffer (25 mM imidazole, 0.02% Tween 80, and 150 mM NaCl, pH 6.5) before the application of the concentrated medium. The concentrated medium was allowed to flow through the column, and the equilibration buffer, first washing buffer (25 mM imidazole, 0.02% Tween 80, and 100 mM sodium phosphate, pH 6.3), and second washing buffer (25 mM imidazole, 0.02% Tween 80, 100 mM sodium phosphate, and 1 M NaCl, pH 6.3) were then allowed to flow through the column to wash off the impurities. When the washing was completed, the fusion protein bound to the column was eluted with an elution buffer (25 mM imidazole, 0.02% Tween 80, and 500 mM sodium phosphate, pH 6.3). The eluate was replaced with Q-equilibration buffer (25 mM Histidine, 0.02% Tween 80, and 25 mM NaCl, pH 6.0) using a diafiltration method, and the elute was applied to a previously packed Hitrap Q HP(1.6×2.5cm, 5 mL) column. Thereafter, the column was washed with Q-equilibration buffer, and the FVII/fusion protein bound to the column was then eluted with Q-elution buffer (25 mM histidine, 0.02% Tween 80, 0.025 mM CaCl₂, and 1 M NaCl, pH 6.0). The eluted protein was analyzed by a FVII-chromogenic assay, FVII ELISA, and SDS-PAGE/Western blotting method.

### <8-2> Western blotting assay

The FVII and FVII/Tf fusion proteins partially purified through the 2-step column were confirmed to have a purity of 45% or more, as measured by SDS-PAGE/Coomassie blue staining. Since the human FVII remaining in an animal test was analyzed using an ELISA method, the presence of FVII-derived fragments in the purified fusion protein was confirmed by Western blotting. NovoSeven^{®} (Novo Nordisk, 1.2 mg/vial, 60 KIU) and the purified sample were prepared at 0.1 IU (FVII activity)/10 µL, and the resulting sample solution was subjected to SDS-PAGE using a NuPage 4-12% bis-Tri gel (Invitrogen). After the electrophoresis was completed, the proteins separates by the SDS-PAGE was transferred to a PVDF membrane, and the membrane was blocked at room temperature for 1 hour in 10 mL with blocking buffer (25 mM Tris, 150 mM NaCl (pH 7.2), 5% skin milk, and 0.1% Tween 80). The blocking solution was discarded, and 10 mL (5% skim milk in PBS-T) of anti-FVII antibodies(Cat. No.: F8146, Sigma) or mouse anti-transferrin antibodies(sc52256, Santa Cruz) was added to the membrane at a ratio of 1:5000 and 1:500, respectively, and the containing the membrane and antibodies solution was stirred for 1 hour. the membrane was washed four times with a washing solution (25 mM Tris, and 150 mM NaCl, pH 7.2) and then stirred for 1 hour in 10 mL (5% skim milk in PBS-T) in a solution supplemented with secondary antibodies, goat anti-mouse IgG-HRP antibody (Cat. No G21040, Invitrogen), at a ratio of 1:50,000. The membrane was washed four times with a washing solution (25 mM Tris, and 150 mM NaCl, pH 7.2), and 2 mL of Super-signal west Femto mix (Thermo) was added to the membrane and reacted for 5 minutes. After the reaction was completed, the film was developed.

The Western blotting results are shown in FIG. 7. As shown in FIG. 7, no FVII-derived fragments was present in the purified protein and noTf-derived fragments was present in the transferrin blot.

### <8-3> Half-life measurement

The half-lives of the five fusion proteins (for example, one fusion protein having no linker and four fusion proteins having the linkers (GS1, GS1-T, GS3, and GS15)) and the half-lives of the two controls (for example, wild-type FVII expressed and purified under the same conditions and commercially available NovoSeven^{®}) were compared in rats. The analysis of samples to be administered and to be acquired were performed by human FVII ELISA (Cedarlane, Paired Antibodies for ELISA Factor VII, #CL20030K) according to the manufacturer's guideline. The concentration of the sample to be administered was determined as an average value of the values measured in triplicate after the sample was diluted with an administration/dilution solution (3 mg/mL of NaCl, 1.5 mg/mL of CaCl₂ dihydrate, 1.3 mg/mL of glycylglycine, 0.1 mg/mL of Polysorbate 80, and 30 mg/mL of mannitol, pH 5.5). After the FVII ELISA quantification, each of the fusion proteins was diluted with the administration/dilution solution and administered intravenously into the tails of rats at a dose of 150 IU/kg, based on the weights of the rats (Sprague-Dawley rats weighing 250 to 300 g) measured on the day of experiments (groups of three rats according to the type of FVII). Blood was drawn a total of 11 times before drug administration, 5 minutes, 15 minutes, 30 minutes, 60 minutes, 1.5 hours, 2 hours, 4 hours, 6 hours, 8 hours, and 24 hours after the drug administration. 225 µL of the drawn blood and 25 µL of 3.2% sodium citrate were mixed and centrifuged at 4°C, 13,000 rpm for 1 minute to obtain supernatant, which was then stored at -70°C. The sample was diluted at a ratio of 1/50 or 1/100 with a washing buffer used in FVII ELISA kit (Cedarlane) and then subjected to rat plasma analysis. A trend curve equation was obtained from the amount of the human FVII antigen remaining at the measured points of time. Finally, the half-lives of the respective test subjects were calculated by the equation 'Half-life = LN(2)/Trend curve slope'. As listed in the following Table 6, it was revealed that the fusion proteins according to one exemplary embodiment of the present invention had the half-life 3 to 4 times higher than the non-fused FVII.

**[Table 6]**

| Kinds of fusion protein | Half-life (min) |
|---|---|
| FVII-GS1-Tf | 254.2 ±19.1 |
| FVII-GS3-Tf | 227.4 ±23.5 |
| FVII-GS1-T-Tf | 235.4 ±27.4 |
| FVII-GS15-Tf | 257.0 ±23.9 |
| FVII-Tf | 277.0 ±24.5 |
| NovoSever^{®} | 80.3 ±27.4 |
| Wild-type FVII | 59.6 ±2.9 |

The FVII-fusion proteins prepared in Examples 2 to 5 were expressed in CHO (VK2) cells that is derived from CHO cell line in which VKORC1 was stably expressed.

The expression vectors constructed in Examples 2 to 5 were purified using Endo-free plasmid maxi kit (Qiagen, #27104). β-Galactosidase was used as the control for transfection. CHO (VK2) cells were seeded in a 6-well plate at a concentration of 1.5×10⁶ cells/well. The cells were cultured for 24 hours in α-MEM (Lonza, #12-169F) supplemented with 10% FBS (Lonza, #14-501F), 1× HT (Invitrogen, #11067-030), 4 mM L-glutamine (Lonza, #17-605E), and 200 µg/mL of hygromycin (Invitrogen, #10687-010), and then the cells were transfected according to the supplier's manual using Lipofectamine 2000 (Invitrogen). After 4 hours of the transfection, the culture medium was replaced with a serum-free culture medium (OptiMEM), and 5 µg/mL of vitamin K was added thereto. After 48 hours of the culture, the culture medium was sampled, and stored at -70°C.

### Example 9: isolation and purification of FVII fusion protein (1)

### <9-1> Expression of FVII fusion protein and isolation/concentration of culture medium

The expression vector including the FVII-GS1-T-Tf fusion protein was expressed in CHO cell line, as described above in Example <8-1>. The transfected cells were cultured at 37°C for 3 to 5 days in a 5% CO₂ incubator, and the cell culture medium was filtered through a 0.22 µm filter (Corning) to remove cells and cell debris remaining in the culture medium. The filtered culture medium was concentrated 10 times by ultrafiltration using a tangential-flow membrane (Satorious, 30 kDa). Substances, such as the FVII fusion proteins, having a high molecular weight of 130 kDa do not pass through the filtration membrane, but substances having a low molecular weight, inorganic salts, or buffer components pass through the porous membrane. The concentrated culture medium was confirmed to have a yield of approximately 90%, compared to the activity (%) of the initial culture medium.

### <9-2> Isolation and purification of fusion protein by affinity chromatography

The culture medium concentrated in Example <9-1> was isolated and purified by affinity chromatography using an XK16/20 VIISelect column (GE Healthcare) packed with a VIISelect resin.

Specifically, prior to performing the chromatography, a VIISelect column was equilibrated with a buffer (pH 7.5) including 50 mM Tris and 150 mM sodium chloride. Thereafter, the column was loaded with the concentrated culture medium, and washed with a buffer (pH 7.5) including 50 mM Tris and 150 mM sodium chloride so as to remove the impurities in the column. After the washing, the FVII fusion protein was eluted using an aqueous 2.5 M sodium thiocyanate buffer (pH 7.0) as the elution buffer.

Using the chromatogram (GE healthcare, Equipment name: AKTA explorer) and SDS-PAGE, it was confirmed that the FVII fusion protein was present in the eluent. The results are shown in FIGS. 8 and 9. FIG. 8 shows the results analyzed by chromatogram in a purification procedure using VIISelect: A, B and C represent an injected culture medium, the impurities not bound to the resin, and the fusion protein eluate, respectively.

Also, FIG. 9 shows the results analyzed using Bio-rad ChemiDoc XRS program image analyzer after the fusion proteins are subjected to SDS-PAGE in a 4-12% Bis-Tris gel under the non-reducing conditions, followed by silver staining: A and C represent an injected culture medium, and the fusion protein eluate, respectively.

As seen from the results, the FVII fusion proteins having a purity of at least 95% or more were obtained in a yield at least 90% using the VIISelect column.

### <9-3> Isolation and purification of the fusion protein by anion exchange chromatography

To isolate active fragments of the FVII fusion proteins, the eluent passed through the VIISelect column in Example <9-2> was subjected to anion exchange chromatography using a column (1.6×2.5 cm, Charge: 5 mL; GE Healthcare) packed with Q-sepharose FF gel, and the active fragment of the FVII fusion protein was further isolated and purified using a method in which protein binds to an amine group via net charge reaction.

Specifically, prior to performing the chromatography, the eluent obtained in Example <9-2> was concentrated and dialyzed with buffer (pH 8.0) including 20 mM Tris, 20 mM sodium chloride, and 5 mM calcium chloride.

Next, column was equilibrated at a flow rate of 5 mL/min using a buffer (pH 8.0) including 20 mM Tris, 20 mM sodium chloride, and 5 mM calcium chloride before the concentrated eluent was loaded onto the Q-sepharose FF column. After eluent was loaded onto the column, fragments of the FVII fusion protein was isolated by a gradient elution method using a buffer (pH 8.0) including 20 mM Tris, 5 mM calcium chloride, and 20 mM to 1 M sodium chloride.

Using the chromatogram and SDS-PAGE, it was confirmed that the FVII fusion protein was present in the eluent. The results are shown in FIGS. 10 and 11.

FIG. 10 shows the results analyzed by chromatogram during a purification procedure using Q-sepharose FF gel: A and B represent an active fusion protein eluate and an inactive fusion protein eluate, respectively. From the chromatogram results, it could be seen that an active form of fragment A was eluted at a sodium chloride concentration of 86.64 mM.

Also, FIG. 11 shows the results analyzed using Bio-rad ChemiDoc XRS program image analyzer after the fusion proteins were subjected to SDS-PAGE in a 4-12% Bis-Tris gel under the reducing conditions, followed by Coomassie blue staining. In FIG. 11, A and B show the results analyzed by quantifying an active fusion protein eluate and an inactive form of the fragment at a concentration of 3 µg, respectively. As seen from the SDS-PAGE results, the active fragments (A) and the inactivated fragments (B) were able to be isolated using the purification method according to one exemplary embodiment of the present invention.

As seen from the results, the FVII fusion proteins having a purity of at least 98% or more were obtained in a yield at least 50% using the Q-sepharose FF column.

### Comparative Example 1: Isolation and purification of FVII fusion protein by affinity chromatography under other elution conditions

The FVII fusion protein was isolated and purified by repeatedly performing the same procedure as in Example <9-2>, except that an elution buffer (pH 7.5) including 50 mM Tris, 1.5 M sodium chloride, and 50% propylene glycol was used instead of the elution buffer (pH 7.0) including 2.5 M sodium thiocyanate upon the isolation and purification using the affinity chromatography in Example <9-2>.

The eluents obtained by the method above were analyzed by chromatogram in the same manner as in Example <9-2> using chromatogram. The results are shown in FIG. 12. In the drawing, A, B, and C represent an injected culture medium, the impurities not bound to the resin, and the fusion protein eluate, respectively.

As seen in FIG. 12, the FVII fusion protein was recovered in a very low yield (5% or less) when the elution buffer (pH 7.5) including 50 mM Tris, 1.5 M sodium chloride, and 50% propylene glycol was used. By comparing the results of Example <9-2> to those of Comparative Example 1, the FVII fusion proteins were isolated and purified more effectively when the aqueous buffer (pH 7.0) including 2.5 M sodium thiocyanate was used in the affinity chromatography, instead of the elution buffer including 50 mM Tris, 1.5 M sodium chloride, and 50% propylene glycol.

### Example 10: Isolation and purification of FVII fusion protein (2)

The procedure in Example 9 was repeatedly performed, except that mixed-mode chromatography to be described below was used instead of the affinity chromatography in Example <9-2>.

### <10-1> Isolation and purification of fusion protein by mixed-mode chromatography

The culture medium concentrated in Example <9-1> was isolated and purified by mixed-mode chromatography using a column (Bio rad) packed with a ceramic fluoroapatite gel (Ca₁₀(PO₄)₆F₂).

Specifically, prior to performing the chromatography, an XK16/20 ceramic fluoroapatite column was equilibrated with a buffer (pH 6.5) including 25 mM imidazole and 0.02% Tween 80. Thereafter, the concentrated culture medium obtained in Example <9-1> was loaded into the column, and the column was primarily washed with a buffer (pH 6.3) including 25 mM imidazole, 0.02% Tween 80, and 100 mM Na-Pi, and then secondarily washed with a buffer including 25 mM imidazole, 0.02% Tween 80, 100 mM Na-Pi, and 0.2 M sodium chloride (pH 6.3) so as to remove the impurities in the column and unwanted isoforms thereof. After the washing, the FVII fusion protein was eluted by a phosphate step gradient method using an elution buffer (pH 6.3) including 25 mM imidazole, 0.02% Tween 80, and 180-340 mM Na-Pi. Using the chromatogram and SDS-PAGE, it was confirmed that the FVII fusion protein was present in the eluent. The results are shown in FIGS. 13 and 14. FIG. 13 shows the results analyzed by chromatogram in a purification procedure using a ceramic fluoroapatite gel: A, B, C, and D represent an injected culture medium, primary washing eluate, secondary washing eluate, and a fusion protein eluate, respectively. Also, FIG. 14 shows the results analyzed using Bio-rad ChemiDoc XRS program image analyzer after the fusion proteins are subjected to SDS-PAGE in a 4-12% Bis-Tris gel under the non-reducing conditions, followed by silver staining: A, B, C, and D represent an injected culture medium, a primary washing eluate, secondary washing eluate, and a fusion protein eluate, respectively.

As seen from the results, the FVII fusion proteins having a purity of at least 90% or more were obtained in a yield at least 60% using the mixed-mode ceramic fluoroapatite column.

### <10-2> Isolation and purification of the fusion protein by anion exchange chromatography

Next, the eluent passed through the ceramic fluoroapatite gel was isolated and purified by the anion exchange chromatography as described in Example <9-3>. The FVII fusion proteins having a purity of at least 98% or more were able to be obtained in a yield at least 50% through the isolation and purification.

### Comparative Example 2: Isolation and purification of FVII fusion protein using ceramic hydroxyapatite column

The procedure in Example <10-1> was repeatedly performed, except that a ceramic hydroxyapatite column to be described below was used instead of the ceramic fluoroapatite column upon the isolation and purification using the mixed-mode chromatography in Example <10-1>.

Specifically, prior to performing the chromatography, an XK16/20 ceramic hydroxyapatite column (Bio-rad) was equilibrated with a buffer (pH 6.5) including 25 mM imidazole, 100 mM sodium chloride, and 0.02% Tween 80. Thereafter, the concentrated culture medium obtained in Example <9-1> was loaded into the column, and the column was primarily washed with a buffer (pH 6.3) including 25 mM imidazole, 0.02% Tween 80, and 100 mM Na-Pi, and then secondarily washed with a buffer (pH 6.3) including 25 mM imidazole, 0.02% Tween 80, 100 mM Na-Pi, and 0.6 M sodium chloride so as to remove the impurities in the column and unwanted isoforms thereof. After the washing, the FVII fusion protein was eluted by a phosphate step gradient method using an elution buffer (pH 6.3) including 25 mM imidazole, 0.02% Tween 80, and 180-340 mM Na-Pi. Using the chromatogram and SDS-PAGE, it was confirmed that the FVII fusion protein was present in the eluent. The results are shown in FIGS. 15 and 16. FIG. 15 shows the results analyzed by chromatogram in a purification procedure using a ceramic hydroxyapatite gel: A, B, C, and D represent injected culture medium, primary washing eluate, secondary washing eluate, and the fusion protein eluate, respectively. Also, FIG. 16 shows the results analyzed using Bio-rad ChemiDoc XRS program image analyzer after the fusion proteins are subjected to SDS-PAGE in a 4-12% Bis-Tris gel under the non-reducing conditions, followed by silver staining: A, B, C, and D represent injected culture medium, primary washing eluate, secondary washing eluate, and the fusion protein eluate, respectively.

As seen from the results, the results obtained by the ceramic hydroxyapatite column showed that the FVII fusion proteins having a purity of at least 90% or more were obtained in a yield at least 52%. As seen from the results, that the yield was reduced, compared to the results obtained through the ceramic fluoroapatite column used in Example <10-1>.

## Claims

1. A method of isolating and purifying a fusion protein comprising factor VII, the method comprising:
1) isolating and purifying a fusion protein comprising factor VII expressed in animal cells by affinity chromatography using a resin having a structure represented by Formula 1 as a stationary phase or mixed-mode chromatography using a ceramic fluoroapatite gel (Ca₁₀(PO₄)₆F₂) as a stationary phase; and
2) further isolating and purifying the fusion protein by anion exchange chromatography using Q-sepharose FF gel as a stationary phase: 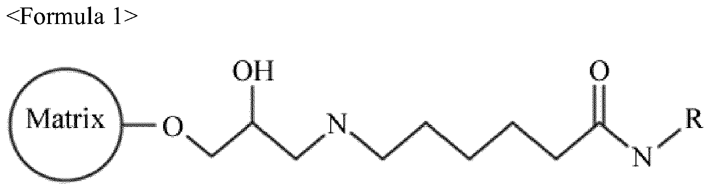 wherein the matrix is cross-linked agarose, and R is a factor VII binding protein serving as a ligand.

2. The method of claim 1, wherein the animal cells are selected from the group consisting of CHO cells, BHK21 cells, HEK293 cells, and Hep G2 cells.

3. The method of claim 1, wherein the fusion protein comprising the factor VII is a protein in which the factor VII is fused to transferrin.

4. The method of claim 3, wherein the fusion protein comprises a linker between the factor VII and the transferrin.

5. The method of claim 4, wherein the linker is a peptide having an amino acid sequence set forth in one of SEQ ID NOs: 3 to 12.

6. The method of claim 1, wherein the affinity chromatography in step 1 uses an aqueous buffer (pH 5.0 to 8.0) containing sodium thiocyanate as an elution buffer.

7. The method of claim 1, wherein the mixed-mode chromatography in step 1 uses an aqueous buffer (pH 5.0 to 8.0) comprising sodium phosphate and sodium chloride.

8. The method of claim 1, wherein the anion exchange chromatography in step 2 uses an aqueous buffer (pH 5.0 to 8.0), which comprises 10 mM to 50 mM Tris, 1 mM to 10 mM calcium chloride, and 50 mM to 150 mM sodium chloride, as an elution buffer.
